(19) Europäisches Patentamt
European Patent Office
Office européen des brevets

(11) EP 4 035 528 B1

(12) EUROPEAN PATENT SPECIFICATION

(45) Date of publication and mention of the grant of the patent:
**27.03.2024 Bulletin 2024/13**

(21) Application number: **20870221.7**

(22) Date of filing: **23.09.2020**

(51) International Patent Classification (IPC):
***A01M 21/04*** *(2006.01)* ***C07D 491/044*** *(2006.01)*
***A01N 43/58*** *(2006.01)* ***A01N 43/90*** *(2006.01)*
***A01P 13/02*** *(2006.01)* ***C07D 519/00*** *(2006.01)*

(52) Cooperative Patent Classification (CPC):
**A01N 43/90; A01P 13/02; C07D 491/044; C07D 519/00**

(86) International application number:
**PCT/JP2020/035689**

(87) International publication number:
**WO 2021/060240 (01.04.2021 Gazette 2021/13)**

(54) **7-OXA-3,4-DIAZABICYCLO[4.1.0]HEPT-4-EN-2-ONE COMPOUND AND HERBICIDE**

7-OXA-3,4-DIAZABICYCLO[4.1.0]HEPT-4-EN-2-ON-VERBINDUNG UND HERBIZID

COMPOSÉ 7-OXA-3,4-DIAZABICYCLO[4.1.0]HEPT-4-ÈNE-2-ONE ET HERBICIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2019 JP 2019174531**

(43) Date of publication of application:
**03.08.2022 Bulletin 2022/31**

(73) Proprietor: **Nippon Soda Co., Ltd.**
**Tokyo 100-7010 (JP)**

(72) Inventors:
- **MIHARA, Ken**
  **Odawara-shi, Kanagawa 250-0280 (JP)**
- **IKEDA, Yoji**
  **Odawara-shi, Kanagawa 250-0280 (JP)**
- **TAKI, Yukina**
  **Odawara-shi, Kanagawa 250-0280 (JP)**
- **KATO, Kazushige**
  **Odawara-shi, Kanagawa 250-0280 (JP)**
- **OOKA, Hirohito**
  **Odawara-shi, Kanagawa 250-0280 (JP)**
- **FUJII, Kazushige**
  **Odawara-shi, Kanagawa 250-0280 (JP)**

(74) Representative: **Wibbelmann, Jobst**
**Wuesthoff & Wuesthoff**
**Patentanwälte und Rechsanwalt PartG mbB**
**Schweigerstrasse 2**
**81541 München (DE)**

(56) References cited:
**EP-A1- 3 438 095** **JP-A- 2014 528 960**
**JP-A- 2017 518 984** **US-A1- 2017 096 402**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 035 528 B1

## Description

BACKGROUND OF THE INVENTION

Field of the Invention

The present invention relates to a 7-oxa-3,4-diazabicyclo [4.1.0] hept-4-en-2-one compound and a herbicide containing the same as an active ingredient.

The present application claims priority on Japanese Patent Application No. 2019-174531, filed in Japan on September 25, 2019.

Description of the Related Art

In the cultivation of agricultural and horticultural crops, herbicides may be used for controlling weeds. Various compounds have been proposed so far as active ingredients of herbicides.

For example, Patent Document 1 discloses a compound represented by a formula (A).

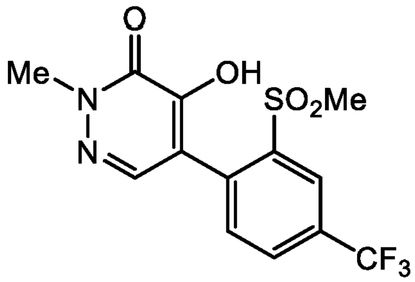

(A)

PRIOR ART DOCUMENTS

[Patent Document]

[Patent Document 1] WO2013 / 050421A

Furthermore EP3438095 A1 or US 2017/0096402 A1 disclose further phenyl-hydroxy(or alkoxy)-pyridazinone derivatives. 1

SUMMARY OF THE INVENTION

Problems to be Solved by the Invention

Herbicides are required not only to have an excellent weed control effect, but also to have less phytotoxicity to crops, to be less likely to remain in the environment, and not to pollute the environment.

An object of the present invention is to provide a novel 7-oxa-3,4-diazabicyclo [4.1.0] hept-4-en-2-one compound useful as an active ingredient of a herbicide, which has a reliable weed control effect even at a low dose, has less phytotoxicity to crops, and is highly safe for the environment; and a herbicide.

Means for Solving the Problem

As a result of intensive studies in order to achieve the above object, the present invention including the following embodiments has been completed.

[1] A compound represented by a formula (I) or a salt thereof.

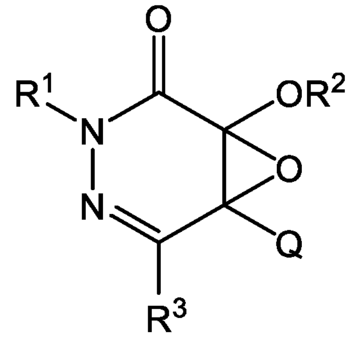

(I)

In the formula (I),

$R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group, or a 5- to 6-membered cyclic ether group,
$R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group,
$R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group, or a substituted or unsubstituted phenyl group, and
Q represents a substituted or unsubstituted phenyl group or a substituted or unsubstituted naphthyl group.

[2] The compound represented by the formula (I) according to the above [1] which is represented by a formula (I-1), or a salt thereof.

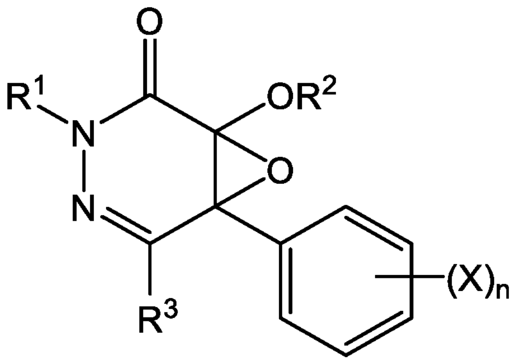

(I-1)

In the formula (I-1), $R^1$, $R^2$, and $R^3$ are the same as defined in the above [1],

X represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a substituted or unsubstituted $C_{2-6}$ alkenyloxy group, a substituted or unsubstituted $C_{2-6}$ alkynyloxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted $C_{3-6}$ cycloalkyloxy group, a substituted or unsubstituted phenyl group, a phenoxy group, a substituted or unsubstituted 5- to 6-membered heterocyclyl group, a substituted or unsubstituted 5- to 6-membered heterocyclyloxy group, a substituted or unsubstituted phenylsulfonyl group, a group represented by R-CO-, a group represented by RO-CO-, a group represented by R-CONR$^a$-, a group represented by RNH-CO-, a group represented by $R_2$N-CO-, a group represented by RO-CO-NR$^a$-, a group represented by RNH-CO-NH-, a group represented by $R_2$N-CO-NH-, a group represented by RNH-CO-CO-NH-, a group represented by $R_2$N-CO-CO-NH-, a group represented by R-S(O)$_2$-NH-, a group represented by $R_2$N-S(O)$_2$-, a group represented by $R_2$S(O)=N-, a group represented by R-S(O)(=N-R$^b$)-, a group represented by RO-N=C(R$^c$)-, a nitro group, or a cyano group;

each R independently represents a substituted or unsubstituted $C_{1-6}$ alkyl group or a substituted or unsubstituted $C_{3-6}$ cycloalkyl group,
each $R^a$ independently represents a hydrogen atom, a substituted or unsubstituted $C_{1-6}$ alkyl group, or a substituted or unsubstituted $C_{1-6}$ alkoxy group,
$R^b$ represents a substituted or unsubstituted $C_{1-6}$ alkyl group or a substituted or unsubstituted phenyl group,
$R^c$ represents a hydrogen atom or a substituted or unsubstituted $C_{1-6}$ alkyl group;
wherein in the above-described group represented by $R_2N$-CO-, the group represented by $R_2N$-CO-NH-, the group represented by $R_2N$-CO-CO-NH-, or the group represented by $R_2N-S(O)_2$-, R and R may be bonded to form a 4- to 6-membered ring together with a nitrogen atom to which they are bonded;
in the above group represented by $R_2S(O)=N$-, R and R may be bonded to form a 5- to 6-membered ring together with a sulfur atom to which they are bonded;
n represents an integer of 0 to 5, when n is 2 or more, the X groups may be the same or different, and when n is 2 or more, two of the X groups thereof may be combined to form a divalent organic group.

[3] The compound represented by the formula (I) according to the above [1] which is represented by a formula (I-3), or a salt thereof.

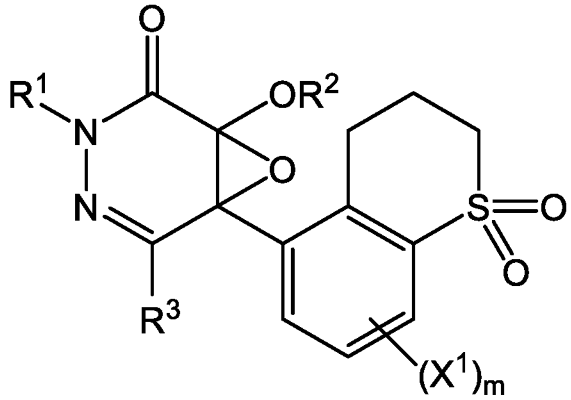

(I-3)

In the formula (I-3), $R^1$, $R^2$, and $R^3$ are the same as defined in the above [1], wherein $X^1$ represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted phenyl group, a substituted or unsubstituted 5- to 6-membered heterocyclyl group, a nitro group, or a cyano group;
m represents an integer of 0 to 3, and when m is 2 or more, the $X^1$ groups may be the same or different.
[4] The compound according to the above [1] or a salt thereof, wherein a substituent on the $C_{1-6}$ alkyl group represented by $R^2$ is at least one selected from the group consisting of a halogeno group, a hydroxyl group, a $C_{1-6}$ alkoxy group, a $C_{1-6}$ alkoxy $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group, a $C_{3-6}$ cycloalkyl group, a phenyl group, a 5-membered heteroaryl group, a $C_{1-6}$ alkylcarbonyl group, a benzoyl group, a $C_{1-6}$ alkoxycarbonyl group, a $C_{1-6}$ alkylcarboxamide group, a (1,3-dioxoisoindrin-2-yl)oxy group, a trimethylsilyl group and a cyano group, and
a group consisting of a $C_{1-6}$ alkyl group substituted, halogeno group substituted, $C_{1-6}$ haloalkyl group substituted or $C_{1-6}$ haloalkoxy group substituted phenyl group; a $C_{1-6}$ alkyl group substituted, halogeno group substituted, $C_{1-6}$ haloalkyl group substituted or $C_{1-6}$ haloalkoxy group substituted phenoxy group; and a $C_{1-6}$ alkyl group substituted, halogeno group substituted, $C_{1-6}$ haloalkyl group substituted or $C_{1-6}$ haloalkoxy group substituted 5-membered heteroaryl group.
[5] A herbicide containing at least one selected from the group consisting of the compound according to any one of the above [1] to [4] and a salt thereof as an active ingredient.
[6] A method for controlling monocotyledonous and / or dicotyledonous weeds in useful plants, which is a method including a step of applying the compound according to any one of the above [1] to [4] or a salt thereof, or a herbicide containing the aforementioned compound to the aforementioned weed and / or the aforementioned plant and / or

its location.

Effects of the Invention

Since the 7-oxa-3,4-diazabicyclo [4.1.0] hept-4-en-2-one compound of the present invention has a reliable weed control effect even at a low dose, has less phytotoxicity to crops, and is highly safe for the environment, it is useful as an active ingredient of a herbicide. The herbicide of the present invention can be safely used for controlling weeds in the cultivation of agricultural and horticultural crops.

DETAILED DESCRIPTION OF THE INVENTION

The 7-oxa-3,4-diazabicyclo [4.1.0] hept-4-en-2-one compound of the present invention (hereinafter, may be referred to as "the compound of the present invention" for simplicity) is a compound represented by the formula (I) (sometimes referred to as compound (I)) or a salt of the compound (I). The compound (I) also includes hydrates, various types of solvates, polymorphs of crystals.
The compound (I) may have stereoisomers based on asymmetric carbons, double bonds and tautomers. All such isomers and mixtures thereof are within the technical scope of the present invention.
The term "unsubstituted" used in the present specification means that it is composed only of a group which becomes a mother nucleus. When it is described only by the name of the group which becomes the mother nucleus without being described as "substituted", it means "unsubstituted" unless otherwise stated.
On the other hand, the term "substituted" means that any hydrogen atom of a group which becomes a mother nucleus is substituted with a group (substituent) having the same or different structure as that of the mother nucleus. Therefore, a "substituent" is another group bonded to a group which becomes a mother nucleus. The number of substituents may be one, or two or more. The two or more substituents may be the same or different.
The terms "$C_{1-6}$" mean that the number of carbon atoms in the group which becomes a mother nucleus is 1 to 6.
The number of carbon atoms does not include the number of carbon atoms present in the substituent. For example, a butyl group having an ethoxy group as a substituent is classified as a $C_2$ alkoxy $C_4$ alkyl group.
A "substituent" is chemically acceptable and is not particularly limited as long as it has the effects of the present invention. Hereinafter, groups which can be a "substituent" are exemplified.
A$C_{1-6}$ alkyl group such as a methyl group, an ethyl group, an n-propyl group, an i-propyl group, an n-butyl group, an s-butyl group, an i-butyl group, a t-butyl group, an n-pentyl group, and an n-hexyl group;

a $C_{2-6}$ alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
a $C_{2-6}$ alkynyl group such as an ethynyl group, a 1-propynyl group, a 2-propynyl group, a 1-butynyl group, a 2-butynyl group, a 3-butynyl group, and a 1-methyl-2-propynyl group;
a $C_{3-6}$ cycloalkyl group such as a cyclopropyl group, a cyclobutyl group, a cyclopentyl group and a cyclohexyl group;
a phenyl group and a naphthyl group;
a phenyl $C_{1-6}$ alkyl group such as a benzyl group and a phenethyl group;
a 3- to 6-membered heterocyclyl group;
a 3- to 6-membered heterocyclyl $C_{1-6}$ alkyl group;
a hydroxyl group;
a $C_{1-6}$ alkoxy group such as a methoxy group, an ethoxy group, an n-propoxy group, an i-propoxy group, an n-butoxy group, an s-butoxy group, an i-butoxy group and a t-butoxy group;
a $C_{2-6}$ alkenyloxy group such as a vinyloxy group, an allyloxy group, a propenyloxy group and a butenyloxy group;
a $C_{2-6}$ alkynyloxy group such as an ethynyloxy group and a propargyloxy group;
a phenoxy group and a naphthoxy group;
a benzyloxy group and a phenethyloxy group;
a 5- to 6-membered heteroaryloxy group such as a thiazolyloxy group and a pyridyloxy group;
a 5- to 6-membered heteroaryl $C_{1-6}$ alkyloxy group such as a thiazolylmethyloxy group and a pyridylmethyloxy group;
a formyl group;
a $C_{1-6}$ alkylcarbonyl group such as an acetyl group and a propionyl group;
a formyloxy group;
a $C_{1-6}$ alkylcarbonyloxy group such as an acetyloxy group and a propionyloxy group;
a benzoyl group;
a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a $C_{1-6}$ alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group and a t-butoxycarbonyloxy group;

a carboxyl group;

a halogeno group such as a fluoro group, a chloro group, a bromo group, and an iodo group;

a $C_{1-6}$ haloalkyl group such as a chloromethyl group, a chloroethyl group, a difluoromethyl group, a trifluoromethyl group, a 2,2,2-trifluoroethyl group, a 1,2-dichloro-n-propyl group and a 1-fluoro-n-butyl group;

a $C_{2-6}$ haloalkenyl group such as a 2-chloro-1-propenyl group and a 2-fluoro-1-butenyl group;

a $C_{2-6}$ haloalkynyl group such as a 4,4-dichloro-1-butynyl group, a 4-fluoro-1-pentynyl group and a 5-bromo-2-pentynyl group;

a $C_{1-6}$ haloalkoxy group such as a difluoromethoxy group, a trifluoromethoxy group, a 2,2,2-trifluoroethoxy group and a 2,3-dichlorobutoxy group;

a $C_{2-6}$ haloalkenyloxy group such as a 2-chloropropenyloxy group and a 3-bromobutenyloxy group;

a $C_{1-6}$ haloalkylcarbonyl group such as a chloroacetyl group, a trifluoroacetyl group and a trichloroacetyl group;

an amino group;

a $C_{1-6}$ alkyl-substituted amino group such as a methylamino group, a dimethylamino group and a diethylamino group;

an anilino group and a naphthylamino group;

a phenyl $C_{1-6}$ alkylamino group such as a benzylamino group and a phenethylamino group;

a formylamino group;

a $C_{1-6}$ alkylcarbonylamino group such as an acetylamino group, a propanoylamino group, a butyrylamino group and an i-propylcarbonylamino group;

a $C_{1-6}$ alkoxycarbonylamino group such as a methoxycarbonylamino group, an ethoxycarbonylamino group, an n-propoxycarbonylamino group and an i-propoxycarbonylamino group;

an unsubstituted or substituted aminocarbonyl group such as an aminocarbonyl group, a dimethylaminocarbonyl group, a phenylaminocarbonyl group and an N-phenyl-N-methylaminocarbonyl group;

an imino $C_{1-6}$ alkyl group such as an iminomethyl group, a (1-imino)ethyl group and a (1-imino)-n-propyl group;

a substituted or unsubstituted N-hydroxyimino $C_{1-6}$ alkyl group such as an N-hydroxy-iminomethyl group, a (1-(N-hydroxy)-imino)ethyl group, a (1-(N-hydroxy)-imino)propyl group, an N-methoxy-iminomethyl group, and a (1-(N-methoxy)-imino)ethyl group;

an aminocarbonyloxy group;

a $C_{1-6}$ alkyl-substituted aminocarbonyloxy group such as an ethylaminocarbonyloxy group and a dimethylaminocarbonyloxy group;

a mercapto group;

a $C_{1-6}$ alkylthio group such as a methylthio group, an ethylthio group, an n-propylthio group, an i-propylthio group, an n-butylthio group, an i-butylthio group, an s-butylthio group and a t-butylthio group;

a $C_{1-6}$ haloalkylthio group such as a trifluoromethylthio group and a 2,2,2-trifluoroethylthio group;

a phenylthio group;

a 5- to 6-membered heteroarylthio group such as a thiazolylthio group and a pyridylthio group;

a $C_{1-6}$ alkylsulfinyl group such as a methylsulfinyl group, an ethylsulfinyl group and a t-butylsulfinyl group;

a $C_{1-6}$ haloalkylsulfinyl group such as a trifluoromethylsulfinyl group and a 2,2,2-trifluoroethylsulfinyl group;

a phenylsulfinyl group;

a 5- to 6-membered heteroarylsulfinyl group such as a thiazolylsulfinyl group and a pyridylsulfinyl group;

a $C_{1-6}$ alkylsulfonyl group such as a methylsulfonyl group, an ethylsulfonyl group and a t-butylsulfonyl group;

a $C_{1-6}$ haloalkylsulfonyl group such as a trifluoromethylsulfonyl group and a 2,2,2-trifluoroethylsulfonyl group;

a phenylsulfonyl group;

a 5- to 6-membered heteroarylsulfonyl group such as a thiazolylsulfonyl group and a pyridylsulfonyl group;

a $C_{1-6}$ alkylsulfonyloxy group such as a methylsulfonyloxy group, an ethylsulfonyloxy group and a t-butylsulfonyloxy group;

a $C_{1-6}$ haloalkylsulfonyloxy group such as a trifluoromethylsulfonyloxy group and a 2,2,2-trifluoroethylsulfonyloxy group;

a tri $C_{1-6}$ alkyl-substituted silyl group such as a trimethylsilyl group, a triethylsilyl group and a t-butyldimethylsilyl group;

a triphenylsilyl group;

a pentafluorosulfanyl group;

a cyano group; and a nitro group.

Further, in these "substituents", any hydrogen atom in the substituent may be substituted with a group having a different structure. Examples of the "substituent" in this case include a $C_{1-6}$ alkyl group, a $C_{1-6}$ haloalkyl group, a $C_{1-6}$ alkoxy group, a $C_{1-6}$ haloalkoxy group, a halogeno group, a cyano group and a nitro group.

Moreover, the above "3- to 6-membered heterocyclyl group" is a 3-membered ring, 4-membered ring, 5-membered ring or 6-membered ring group containing 1 to 4 hetero atoms selected from the group consisting of a nitrogen atom, an oxygen atom and a sulfur atom as constituent atoms of the ring. The heterocyclyl group may be either monocyclic or polycyclic. As long as the polycyclic heterocyclyl group includes at least one heterocyclic ring, the remaining ring may be a saturated alicyclic ring, an unsaturated alicyclic ring or an aromatic ring. Examples of the "3- to 6-membered heterocyclyl group" include a 3- to 6-membered saturated heterocyclyl group, a 5- to 6-membered unsaturated heterocyclyl group and a 5- to 6-membered heteroaryl group.

Examples of the 3- to 6-membered saturated heterocyclyl group include an aziridinyl group, an epoxy group, an azetidinyl group, a pyrrolidinyl group, a tetrahydrofuranyl group, a dioxolanyl group, a tetrahydropyranyl group, a piperidyl group, a piperazinyl group, a morpholinyl group and a dioxanyl group.

Examples of the 5- to 6-membered unsaturated heterocyclyl group includes a pyrrolinyl group, a dihydrofuranyl group, an imidazolinyl group, a pyrazolinyl group, an oxazolinyl group, an isooxazolinyl group, a thiazolinyl group, an isothiazolinyl group, a dihydropyranyl group and a dihydrooxadinyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridazinyl group and a triazinyl group.

[$R^1$]

$R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group, or a 5 to 6-membered cyclic ether group.

The "$C_{1-6}$ alkyl group" represented by $R^1$ may be a straight chain or a branched chain. Examples of the "$C_{1-6}$ alkyl group" represented by $R^1$ include a methyl group, an ethyl group, an n-propyl group, an n-butyl group, an n-pentyl group, an n-hexyl group, an i-propyl group, an i-butyl group an s-butyl group, a t-butyl group, an i-pentyl group, a neopentyl group, a 2-methylbutyl group and an i-hexyl group.

Examples of the "$C_{2-6}$ alkenyl group" represented by $R^1$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group.

Examples of the "$C_{2-6}$ alkynyl group" represented by $R^1$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

The substituent on the "$C_{1-6}$ alkyl group", "$C_{2-6}$ alkenyl group", or "$C_{2-6}$ alkynyl group" represented by $R^1$ is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; or a cyano group.

Examples of the "$C_{3-6}$ cycloalkyl group" represented by $R^1$ include a cyclopropyl group, a cyclobutyl group, a cyclopentyl group and a cyclohexyl group.

Examples of the "5- to 6-membered cyclic ether group" represented by $R^1$ include a tetrahydrofuranyl group and a tetrahydropyranyl group.

The substituent on the "$C_{3-6}$ cycloalkyl group" represented by $R^1$ is preferably a halogeno group such as a fluoro group, a chloro group, a bromo group, an iodo group; a $C_{1-6}$ alkyl group such as a methyl group, an ethyl group, an n-propyl group, an i-propyl group, an n-butyl group, an s-butyl group, an i-butyl group, a t-butyl group, a n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy

group; a $C_{1-6}$ haloalkoxy group such as a 2,3-dichlorobutoxy group, a trifluoromethoxy group and a 2,2,2-trifluoroethoxy group; or a cyano group.

In the present invention, $R^1$ is preferably a substituted or unsubstituted $C_{1-6}$ alkyl group or a 5 to 6 member cyclic ether group.

The substituent on the "$C_{1-6}$ alkyl group" represented by $R^1$ is preferably a halogeno group, a $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group or a $C_{3-6}$ cycloalkyl group.

[R2]

$R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group. Specific examples of the "unsubstituted $C_{1-6}$ alkyl group", "substituted or unsubstituted $C_{2-6}$ alkenyl group", or "substituted or unsubstituted $C_{2-6}$ alkynyl group" represented by $R^2$ include the same as those exemplified for $R^1$.

The substituent on the "$C_{1-6}$ alkyl group" represented by $R^2$ is preferably a halogeno group such as a fluoro group, a chloro group, a bromo group and an iodo group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkoxy $C_{1-6}$ alkoxy group such as a methoxyethoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; a $C_{1-6}$ alkyl group substituted, halogeno group substituted, $C_{1-6}$ haloalkyl group substituted or $C_{1-6}$ haloalkoxy group substituted phenoxy group; a 5-membered heteroaryl group; a $C_{1-6}$ alkyl group substituted, halogeno group substituted, $C_{1-6}$ haloalkyl group substituted or $C_{1-6}$ haloalkoxy group substituted 5-membered heteroaryl group; a $C_{1-6}$ alkylcarbonyl group such as an acetyl group; a benzoyl group; a $C_{1-6}$ alkoxycarbonyl group such as a methoxycarbonyl group; a $C_{1-6}$ alkylcarboxyamide group such as an acetamide group; a (1,3-dioxoisoindrin-2-yl)oxy group, a trimethylsilyl group or a cyano group.

The "5-membered heteroaryl group" mentioned as one of the substituents on the "$C_{1-6}$ alkyl group" represented by $R^2$ is a group of 5-membered aromatic rings containing 1 to 4 hetero atoms selected from the group consisting of a nitrogen atom, an oxygen atom and a sulfur atom as constituent atoms of the ring.

Examples of the 5-membered heteroaryl group include a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group and a tetrazolyl group.

[R3]

$R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group or a substituted or unsubstituted phenyl group.

Specific examples of these groups represented by $R^3$ include the same as those exemplified for $R^1$.

Examples of the "$C_{1-6}$ alkoxy group" represented by $R^3$ include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group and an i-hexyloxy group.

The substituent on the "$C_{1-6}$ alkoxy group" represented by $R^3$ is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; or a cyano group.

The substituent on the "phenyl group" represented by $R^3$ is preferably a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; or a cyano group.

In the present invention, $R^3$ is preferably a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, or a substituted or unsubstituted $C_{3-6}$ cycloalkyl group.

The substituent on the "$C_{1-6}$ alkyl group", "$C_{2-6}$ alkenyl group", or "$C_{2-6}$ alkynyl group" represented by $R^3$ is preferably a halogeno group. The substituent on the "$C_{3-6}$ cycloalkyl group" is preferably a halogeno group or a $C_{1-6}$ alkyl group.

[Q]

Q represents a substituted or unsubstituted phenyl group or a substituted or unsubstituted naphthyl group.

The substituent on the "phenyl group" or "naphthyl group" represented by Q (sometimes referred to as substituent (X)) is at least one selected from the group consisting of a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a substituted or unsubstituted $C_{2-6}$ alkenyloxy group, a substituted or unsubstituted $C_{2-6}$ alkynyloxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted $C_{3-6}$ cycloalkyloxy group, a substituted or unsubstituted phenyl group, a phenoxy group, a substituted or unsubstituted 5- to 6-membered heterocyclyl group, a substituted or unsubstituted 5- to 6-membered heterocyclyloxy group, a substituted or unsubstituted phenylsulfonyl group, a group represented by R-CO-, a group represented by RO-CO-, a group represented by R-CONR$^a$-, a group represented by RNH-CO-, a group represented by $R_2N$-CO-, a group represented by RO-CO-NR$^a$-, a group represented by RNH-CO-NH-, a group represented by $R_2N$-CO-NH-, a group represented by RNH-CO-CO-NH-, a group represented by $R_2N$-CO-CO-NH-, a group represented by R-$S(O)_2$-NH-, a group represented by $R_2N$-$S(O)_2$-, a group represented by $R_2S(O)$=N-, a group represented by R-S(O)(=N-R$^b$)-, a group represented by RO-N=C(R$^c$)-, a nitro group and a cyano group.

Here, R groups each independently represent a substituted or unsubstituted $C_{1-6}$ alkyl group or a substituted or unsubstituted $C_{3-6}$ cycloalkyl group.

$R^a$ groups each independently represent a hydrogen atom, a substituted or unsubstituted $C_{1-6}$ alkyl group, or a substituted or unsubstituted $C_{1-6}$ alkoxy group.

$R^b$ represents a substituted or unsubstituted $C_{1-6}$ alkyl group or a substituted or unsubstituted phenyl group.

$R^c$ represents a hydrogen atom or a substituted or unsubstituted $C_{1-6}$ alkyl group.

Further, in the above-mentioned group represented by $R_2N$-CO-, the group represented by $R_2N$-CO-NH-, the group represented by $R_2N$-CO-CO-NH-, or the group represented by $R_2N$-$S(O)_2$-, R and R may be bonded to form a 4- to 6-membered ring together with the nitrogen atom to which they are bonded.

Moreover, in the above group represented by $R_2S(O)$=N-, R and R may be bonded to form a 5- to 6-membered ring together with the sulfur atom to which they are bonded.

When there are two or more substituents (X) of the "phenyl group" or "naphthyl group" represented by Q, two of these may be combined to form a divalent organic group.

[X]

X represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a substituted or unsubstituted $C_{2-6}$ alkenyloxy group, a substituted or unsubstituted $C_{2-6}$ alkynyloxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted $C_{3-6}$ cycloalkyloxy group, a substituted or unsubstituted phenyl group, a phenoxy group, a substituted or unsubstituted 5- to 6-membered heterocyclyl group, a substituted or unsubstituted 5- to 6-membered heterocyclyloxy group, a substituted or unsubstituted phenylsulfonyl group, a group represented by R-CO-, a group represented by RO-CO-, a group represented by R-CONR$^a$-, a group represented by RNH-CO-, a group represented by $R_2N$-CO-, a group

represented by RO-CO-$NR^a$-, a group represented by RNH-CO-NH-, a group represented by $R_2N$-CO-NH-, a group represented by RNH-CO-CO-NH-, a group represented by $R_2N$-CO-CO-NH-, a group represented by R-$S(O)_2$-NH-, a group represented by $R_2N$-$S(O)_2$-, a group represented by $R_2S(O)$=N-, a group represented by R-S(O)(=N-$R^b$)-, a group represented by RO-N = C($R^c$)-, a nitro group or a cyano group.

Examples of the "halogeno group" represented by X include a fluoro group, a chloro group, a bromo group and an iodo group.

The "$C_{1-6}$ alkyl group" represented by X may be a straight chain or a branched chain. Examples of the "$C_{1-6}$ alkyl groups" represented by X include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group and an i-hexyl group.

Examples of the "$C_{2-6}$ alkenyl group" represented by X include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group.

Examples of the "$C_{2-6}$ alkynyl group" represented by X include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

Examples of the "$C_{1-6}$ alkoxy group" represented by X include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group and an i-hexyloxy group.

Examples of the "$C_{2-6}$ alkenyloxy group" represented by X include a vinyloxy group, an allyloxy group, a propenyloxy group and a butenyloxy group.

Examples of the "$C_{2-6}$ alkynyloxy group" represented by X include an ethynyloxy group and a propargyloxy group.

Examples of the "$C_{1-6}$ alkylthio group" represented by X include a methylthio group, an ethylthio group, an n-propylthio group, an n-butylthio group, an n-pentylthio group, an n-hexylthio group and an i-propylthio group.

Examples of the "$C_{1-6}$ alkylsulfinyl group" represented by X include a methylsulfinyl group, an ethylsulfinyl group and a t-butylsulfinyl group.

Examples of the "$C_{1-6}$ alkylsulfonyl group" represented by X include a methylsulfonyl group, an ethylsulfonyl group and a t-butylsulfonyl group.

The substituent on the "$C_{1-6}$ alkyl group" or "$C_{1-6}$ alkoxy group" represented by X is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkoxy $C_{1-6}$ alkoxy group such as a methoxyethoxy group; a $C_{3-6}$ cycloalkyl $C_{1-6}$ alkoxy group such as a cyclopropylmethoxy group; a $C_{1-6}$ haloalkoxy group such as a 2,3-dichlorobutoxy group, a trifluoromethoxy group, a 2,2,2-trifluoroethoxy group and a 3,3,3-trifluoropropoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; a morpholinyl group; a 5-membered heteroaryl group such as a triazolyl group; a $C_{1-6}$ alkyl group substituted, halogeno group substituted, $C_{1-6}$ haloalkyl group substituted or $C_{1-6}$ haloalkoxy group substituted 5-membered heteroaryl group; a $C_{1-6}$ alkyl group substituted aminocarbonyl group such as a methylaminocarbonyl group and a dimethylaminocarbonyl group; a (propane-2-ylideneamide)oxy group; or a cyano group.

The substituent on the "$C_{2-6}$ alkenyl group", "$C_{2-6}$ alkynyl group", "$C_{2-6}$ alkynyloxy group", "$C_{1-6}$ alkylthio group", "$C_{1-6}$ alkylsulfinyl group", or "$C_{1-6}$ alkylsulfonyl group" represented by X is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy substituted phenyl group such as a 4-methylphenyl group, a 4-chlorophenyl group, a 4-trifluoromethylphenyl group and a 4-trifluoromethoxyphenyl group; or a cyano group.

Examples of the "$C_{3-6}$ cycloalkyl group" represented by X include a cyclopropyl group, a cyclobutyl group, a cyclopentyl group and a cyclohexyl group.

Examples of the "$C_{3-6}$ cycloalkyloxy group" represented by X include a cyclopropyloxy group, a cyclobutyloxy group, a cyclopentyloxy group, and a cyclohexyloxy group.

The "5- to 6-membered heterocyclyl group" represented by X is a 5-membered ring or 6-membered ring group containing 1, 2, 3 or 4 hetero atoms selected from the group consisting of a nitrogen atom, an oxygen atom and a sulfur atom as ring constituent atoms. When there are two or more hetero atoms, they may be the same or different. Examples of the "5- to 6-membered heterocyclyl group" include a 5- to 6-membered saturated heterocyclyl group, a 5- to 6-membered unsaturated heterocyclyl group, and a 5- to 6-membered heteroaryl group.

Examples of the 5- to 6-membered saturated heterocyclyl group include a pyrrolidinyl group, a tetrahydrofuranyl group, a dioxolanyl group, a tetrahydropyranyl group, a piperidyl group, a piperazinyl group, a morpholinyl group and a dioxanyl group. Examples of the 5- to 6-membered unsaturated heterocyclyl group include a pyrrolinyl group, a dihydrofuranyl group, an imidazolinyl group, a pyrazolinyl group, an oxazolinyl group, an isooxazolinyl group, a thiazolinyl group, an isothiazolinyl group, a dihydropyranyl group and a dihydrooxadinyl group.

Examples of the 5-membered heteroaryl group include a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group and a tetrazolyl group.

Examples of the 6-membered heteroaryl group include a pyridyl group, a pyrazinyl group, a pyrimidinyl group, a pyridadinyl group and a triazinyl group.

The "5- to 6-membered heterocyclyloxy group" represented by X has a structure in which a 5- to 6-membered heterocyclyl group and an oxy group are bonded. Specific examples thereof include a thiazolyloxy group and a pyridyloxy group.

The substituent on the "$C_{3-6}$ cycloalkyl group", "$C_{3-6}$ cycloalkyloxy group", "phenyl group", "phenoxy group", "5- to 6-membered heterocyclyl group", "5- to 6-membered heterocyclyloxy group" or "phenylsulfonyl group" represented by X is preferably a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; an oxo group; or a cyano group.

Specific examples of these groups represented by R, $R^a$, $R^b$, or $R^c$ include the same as those exemplified for X.

Examples of the "group represented by R-CO-" represented by X include an acetyl group and a cyclopropylcarbonyl group.

Examples of the "group represented by RO-CO-" represented by X include a methoxycarbonyl group.

Examples of the "group represented by R-CONR$^a$-" represented by X include an acetamide group and a cyclopropanecarboxyamide group.

Examples of the "group represented by RNH-CO-" represented by X include a methylaminocarbonyl group.

Examples of the "group represented by $R_2$N-CO-" represented by X include a dimethylaminocarbonyl group.

Here, R and R may be bonded to form a 4- to 6-membered ring together with the nitrogen atom to which they are bonded, and examples of the 4- to 6-membered ring to be formed include an azetidine ring, a pyrrolidine ring, a piperidine group, a piperazine ring, and a morpholine ring.

Examples of the "group represented by $R_2$N-CO-" after forming a 4- to 6-membered ring include an azetidine-1-carbonyl group, a pyrrolidine-1-carbonyl group and a morpholin-4-carbonyl group.

Examples of the "group represented by RO-CO-NR$^a$-" represented by X include a (t-butoxycarbonyl) amino group and a methoxy (t-butoxycarbonyl) amino group.

Examples of the "group represented by RNH-CO-NH-" represented by X include methylaminocarboxyamide group.

Examples of the "group represented by $R_2$N-CO-NH-" represented by X include dimethylaminocarboxyamide group.

Here, R and R may be bonded to form a 4- to 6-membered ring together with the nitrogen atom to which they are bonded, and specific examples of the 4- to 6-membered ring to be formed include the same as those exemplified above for the "group represented by $R_2$N-CO-".

Examples of the "group represented by $R_2$N-CO-NH-" after forming a 4- to 6-membered ring include an azetidine-1-carboxyamide group, a pyrrolidine-1-carboxyamide group and a morpholin-4-carboxyamide group.

Examples of the "group represented by RNH-CO-CO-NH-" represented by X include methylaminocarbonylcarboxyamide group.

Examples of the "group represented by $R_2$N-CO-CO-NH-" represented by X include dimethylaminocarbonylcarboxyamide group.

Here, R and R may be bonded to form a 4- to 6-membered ring together with the nitrogen atom to which they are bonded, and specific examples of the 4- to 6-membered ring to be formed include the same as those exemplified above for the "group represented by $R_2N$-CO-".

Examples of the "group represented by $R_2N$-CO-NH-" after forming a 4- to 6-membered ring include an azetidine-1-carbonylcarboxyamide group, a pyrrolidine-1-carbonylcarboxyamide group and morpholin-4-carbonylcarboxyamide group.

Examples of the "group represented by R-S$(O)_2$-NH-" represented by X include methyl sulfonamide group.

Examples of the "group represented by $R_2N$-S$(O)_2$-" represented by X include dimethylaminosulfonyl group.

Here, R and R may be bonded to form a 4- to 6-membered ring together with the nitrogen atom to which they are bonded, and specific examples of the 4- to 6-membered ring to be formed include the same as those exemplified above for the "group represented by $R_2N$-CO-".

Examples of the "group represented by $R_2N$-S$(O)_2$-" after forming a 4- to 6-membered ring include an azetidine-1-sulfonyl group, a pyrrolidine-1-sulfonyl group and a morpholinosulfonyl group.

Examples of the "group represented by $R_2S$(O)=N-" represented by X include a (dimethyloxide-$\lambda^4$-sulfanilidene) amino group ($Me_2S$ (O) = N-).

Here, R and R may be bonded to form a 5- to 6-membered ring together with the sulfur atom to which they are bonded, and examples of the 5- to 6-membered ring to be formed include a tetrahydrothiophene ring and a tetrahydro-2H-thiopyran ring.

Examples of the "group represented by $R_2S$(O)=N-" after forming a 5- to 6-membered ring include a (tetrahydro-1-oxide-2H-thiopyran-1-ylidene) amino group.

Examples of the "group represented by R-S(O)(=N-$R^b$)-" represented by X include an S-methyl-N-methylsulfonimidoyl group and an S-methyl-N-(p-chlorophenyl) sulfonimidoyl group.

Examples of the "group represented by RO-N=C($R^c$)-" represented by X include a (methoxyimino) methyl group and a 1-(methoxyimino) ethyl group.

A "divalent organic group that two X groups may form together" is a substituted or unsubstituted saturated divalent hydrocarbon group having 1 to 4 carbon atoms; or a divalent group formed by bonding a group containing one or more hetero atoms selected from the group consisting of O, N and S, and a substituted or unsubstituted saturated divalent hydrocarbon group having 1 to 4 carbon atoms.

Furthermore, the divalent organic group that two X groups may form together is a substituted or unsubstituted unsaturated divalent hydrocarbon group having 2 to 3 carbon atoms; or a divalent group formed by bonding a group containing one or more hetero atoms selected from the group consisting of O, N and S, and a substituted or unsubstituted unsaturated divalent hydrocarbon group having 2 to 3 carbon atoms.

Examples of the saturated divalent hydrocarbon group having 1 to 4 carbon atoms include a "$C_{1-4}$ alkylene group" such as a methylene group, a dimethylene group, a trimethylene group and a tetramethylene group.

Examples of the unsaturated divalent hydrocarbon group having 2 to 3 carbon atoms include a "$C_{2-3}$ alkenylene group" such as a vinylene group (-CH=CH-) and a propenylene group (-CH=CH-$CH_2$-, -$CH_2$-CH=CH-).

The substituent on the "saturated or unsaturated divalent hydrocarbon group" is preferably a halogen group, a $C_{1-6}$ alkyl group, or a $C_{1-6}$ haloalkyl group.

Examples of the group containing an oxygen atom (O) include an oxy (-O-) group, a carbonyl (-C(O)-) group, and a carboxy (-COO-) group.

As a divalent group formed by bonding an oxygen atom (O) -containing group and a saturated divalent hydrocarbon group, an "oxy $C_{2-3}$ alkylene group" such as an oxydimethylene group (-O-$CH_2CH_2$-), a "$C_{2-3}$ alkyleneoxy group" such as a dimethyleneoxy group (-$CH_2CH_2$-O-), an "oxy$C_{1-2}$ alkyleneoxy group" such as an oxymethyleneoxy group (-O-$CH_2$-O-), a "$C_{1-2}$ alkyleneoxy $C_1$-$C_2$ alkylene group" such as a methyleneoxy methylene group (-$CH_2$-O-$CH_2$-), a methyleneoxydimethylene group (-$CH_2$-O-$CH_2CH_2$-) and a dimethyleneoxymethylene group (-$CH_2CH_2$-O-$CH_2$-).

Examples of the group containing a nitrogen atom (N) include an imino (-NH-) group, an N-substituted imino group, an iminooxy (-NH-O-) group, an N-substituted iminooxy group, an oxyimino (-O-NH-) group, and an N-substituted oxyimino group.

As a divalent group formed by bonding a group containing a nitrogen atom (N) and a saturated divalent hydrocarbon group, an "imino $C_{2-3}$ alkylene group" such as an iminodimethylene group (-NH-$CH_2CH_2$-), a "$C_{2-3}$ alkyleneimino group" such as a dimethyleneimino group (-$CH_2CH_2$-NH-), an "imino $C_{1-2}$ alkyleneimino group" such as an iminomethyleneimino group (-NH-$CH_2$-NH-), a "$C_{1-2}$ alkyleneimino $C_1$-$C_2$ alkylene group" such as a methyleneiminomethylene group (-$CH_2$-NH-$CH_2$-), a methyleneiminodimethylene group (-$CH_2$-NH-$CH_2CH_2$-) and a dimethyleneiminomethylene group (-$CH_2CH_2$-NH-$CH_2$-).

Examples of the group containing a sulfur atom (S) include a thio (-S-) group, a sulfinyl (-S(O)-) group, and a sulfonyl (-S$(O)_2$-) group.

Examples of the divalent group formed by bonding a group containing a sulfur atom (S) and a saturated divalent

hydrocarbon group include a "thio $C_{2-4}$ alkylene group" such as a thiodimethylene group ($-S-CH_2CH_2-$) and a thiotrimethylene group ($-S-CH_2CH_2CH_2-$), a "$C_{2-4}$ alkylene thio group" such as a dimethylene thio group ($-CH_2CH_2-S-$), a "sulfinyl-$C_{2-4}$ alkylene group", a "sulfonyl-$C_{2-4}$ alkylene group", a "$C_{2-4}$ alkylene-sulfinyl group" and a "$C_{2-4}$ alkylene-sulfonyl group".

In addition, an "oxy $C_{1-2}$ alkylene thio group", a "thio $C_{1-2}$ alkylene oxy group", an "oxy $C_{1-2}$ alkylene sulfonyl group", a "sulfonyl $C_{1-2}$ alkylene oxy group" can be mentioned.

In the present invention, the "divalent organic group that two X groups may form together" is preferably a substituted or unsubstituted saturated divalent hydrocarbon group having 1 to 4 carbon atoms; or a divalent group formed by bonding a group containing one or more hetero atoms selected from the group consisting of O and S and a substituted or unsubstituted saturated divalent hydrocarbon group having 1 to 4 carbon atoms. The group containing an oxygen atom (O) is preferably an oxy (-O-) group, and the group containing a sulfur atom (S) is preferably a thio (-S-) group or a sulfonyl ($-S(O)_2-$) group.

In the present invention, Q is preferably a substituted or unsubstituted phenyl group. The compound (I) when Q is a substituted or unsubstituted phenyl group is shown in a formula (I-1).

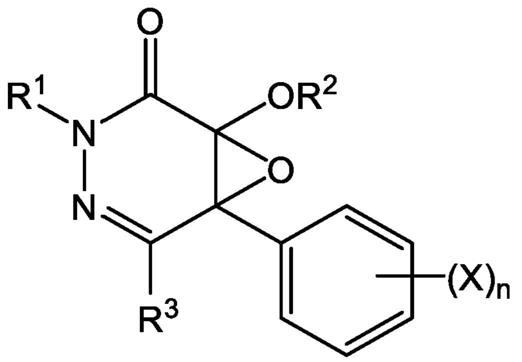

(I-1)

In the formula (I-1), $R^1$ to $R^3$ have the same meanings as those defined in the formula (I). X represents a substituent on the phenyl group mentioned above. n represents an integer from 0 to 5. When n is 2 or more, X may be the same or different. When n is 2 or more, two X groups thereof may be combined to form a divalent organic group.

In the present invention, the compound represented by the above formula (I-1) is preferably a compound represented by the following formula (I-3).

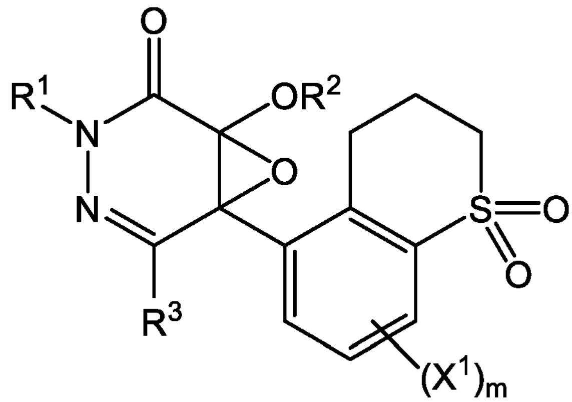

(I-3)

In the formula (I-3), $R^1$ to $R^3$ have the same meanings as those defined in the formula (I).

$X^1$ represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted phenyl group, a substituted or unsubstituted 5- to 6-membered heterocyclyl group, a nitro group, or a cyano group.

m represents an integer from 0 to 3. When m is 2 or more, $X^1$ groups may be the same or different.

Specific examples of these groups represented by $X^1$ include the same as those exemplified for X.

The substituent on the "$C_{1-6}$ alkyl group", "$C_{2-6}$ alkenyl group", "$C_{2-6}$ alkynyl group", "$C_{1-6}$ alkoxy group", "$C_{1-6}$ alkylthio group", "$C_{1-6}$ alkylsulfinyl group" or "$C_{1-6}$ alkylsulfonyl group" represented by $X^1$ is preferably a halogeno group. The substituent on the "$C_{3-6}$ cycloalkyl group" is preferably a halogeno group or a $C_{1-6}$ alkyl group. The substituent on the "phenyl group" or "5-6-membered heterocyclyl group" is preferably a halogeno group, a $C_{1-6}$ alkyl group, a $C_{1-6}$ haloalkyl group, a $C_{1-6}$ alkoxy group, a $C_{1-6}$ haloalkoxy group, or a cyano group.

[Salt]

Examples of a salt of the compound (I) include salts of alkali metals such as lithium, sodium and potassium; salts of alkaline earth metals such as calcium and magnesium; salts of transition metals such as iron and copper; ammonium salts; and salts of organic bases such as triethylamine, tributylamine, pyridine and hydrazine.

The structure of the compound (I) or the salt of the compound (I) can be determined by NMR spectra, IR spectra and MS spectra.

The compound (I) is not particularly limited depending on the production method thereof. Further, the salt of compound (I) can be obtained from the compound (I) by a known method. The compound (I) can be produced, for example, by the method described in Examples, using the compound obtained by the production method described in Patent Document 1 as a production intermediate thereof.

(Reaction scheme 1)

For example, the compound (I) can be prepared from a compound of a formula (2) as shown in the following Reaction Scheme 1. The symbols in the formula (2) have the same meanings as those defined in the formula (I).

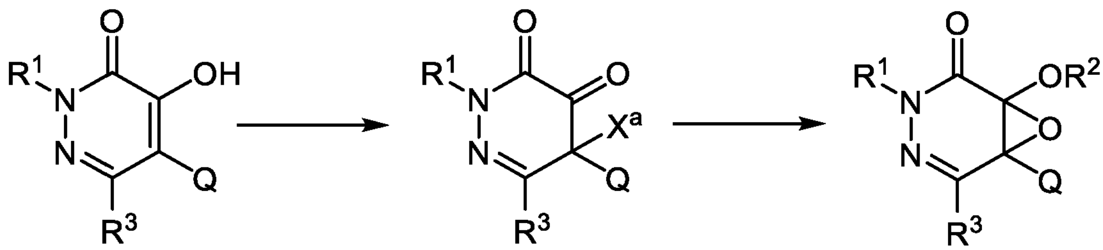

( 2 ) ( 2Xa ) ( I )

The compound of the formula (I) can be prepared by reacting the compound of the formula (2) with a halogenating agent to construct an α-haloketone structure in the molecule, preparing a compound of a formula (2Xa), and subsequently reacting with an alkoxide such as $R_2ONa$ (corresponds to sodium methoxide when $R^2$ is a methyl group). Xa in the formula (2Xa) represents a halogeno group such as a chloro group or a bromo group. The compound of the formula (2Xa) may be unstable, and it is preferable to carry out the subsequent reaction without isolation.

(Reaction scheme 2)

The compound of the formula (2) can be prepared from a compound of a formula (3) as shown in the following reaction scheme 2. The symbols in the formula (3) have the same meanings as those defined in the formula (I). $R^x$ represents a lower alkyl group, such as a methyl group. Hereinafter, $R^x$ has the same meaning as defined above.

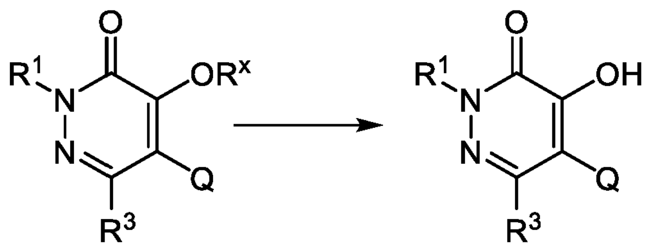

( 3 ) ( 2 )

The compound of the formula (2) can be prepared by heating the compound of the formula (3) with morpholine.

(Reaction scheme 3)

The compound of the formula (3) can be prepared by the condensation of a compound of a formula (4) with a compound of a formula (5) as shown in the following reaction scheme 3.

The symbols in the formula (4) have the same meanings as those defined in the formula (I). $R^y$ represents a lower alkyl group such as a methyl group or an ethyl group. Further, $R^y$ groups may be bonded to each other to form a 1,3,2-dioxaborolane ring. Q in the formula (5) has the same meaning as Q in the formula (I). $X^b$ represents a halogeno group.

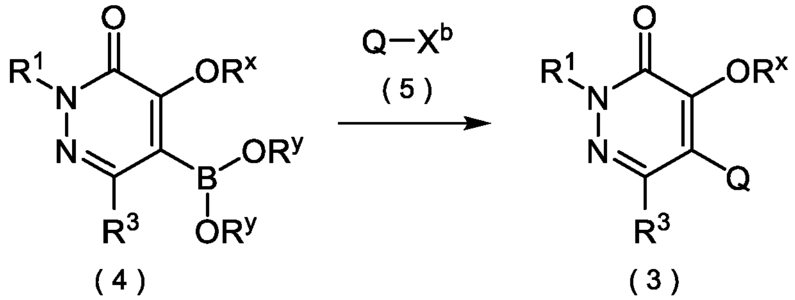

The compounds of the formula (3) can be prepared by reacting the compound of the formula (4) with the compound of the formula (5) in the presence of a suitable base (for example, an inorganic base such as potassium phosphate or cesium fluoride), a metal catalyst (for example, a palladium catalyst such as $Pd(OAc)_2$), and a ligand (for example, a phosphine ligand) according to circumstances.

The metal catalyst and ligand may be added as a preformed complex (for example, a palladium / phosphine complex such as bis (triphenylphosphine) palladium dichloride or a [1,1-bis (diphenylphosphino) ferrocene] palladium dichloride dichloromethane adduct).

Q in the compound of the formula (5) represents a substituted or unsubstituted phenyl group or a substituted or unsubstituted naphthyl group, but the substituent on the phenyl group and the naphthyl group may be appropriately converted even after the reaction.

(Reaction scheme 4)

The compound of the formula (4) can be prepared from the compound of the formula (6) as shown in the following reaction scheme 4. The symbols in the formula (6) have the same meanings as those defined in the formula (I).

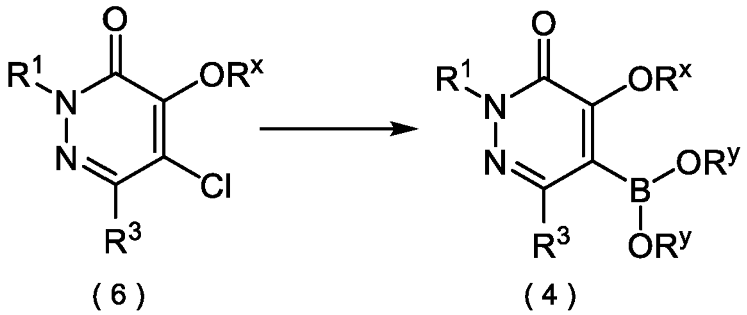

The compounds of the formula (4) can be prepared by reacting the compound of the formula (6) with boronic acid or an ester of boronic acid, such as bis (pinacolato) diboron, in the presence of a suitable base (for example, an inorganic base such as potassium phosphate or cesium fluoride), a metal catalyst (for example, a palladium catalyst such as $Pd_2(dba)_3$ and $(Pd(OAc)_2)$) and a ligand (for example, a phosphine ligand) according to circumstances.

The metal catalyst and ligand may be added as a preformed complex (for example, a palladium / phosphine complex such as bis (triphenylphosphine) palladium dichloride or a [1,1-bis (diphenylphosphino) ferrocene] palladium dichloride dichloromethane adduct).

(Reaction scheme 5)

The compound of the formula (6) can be prepared from the compound of the formula (7) as shown in the following reaction scheme 5. The symbols in the formula (7) have the same meanings as those defined in the formula (I).

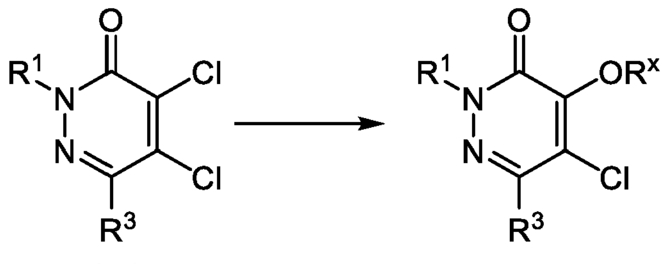

(7) (6)

The compound of the formula (6) can be prepared by reacting the compound of the formula (7) with a suitable metal alkoxide such as sodium methoxide.

The compound of the formula (7) can be prepared by a known method.

(Reaction scheme 3A)

The compound of the formula (3) can be prepared by the condensation of the compound of the formula (6) with the compound of the formula (8) as shown in the following reaction scheme 3A.

Q in the formula (8) has the same meaning as Q in the formula (I). $R^y$ represents a lower alkyl group such as a methyl group or an ethyl group. Further, $R^y$ groups may be bonded to each other to form a 1,3,2-dioxaborolane ring.

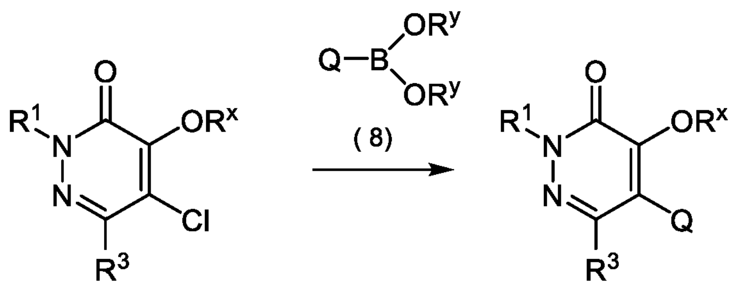


(6) (3)

The compound of the formula (3) can be prepared by reacting the compound of the formula (6) with the compound of the formula (8) in the presence of a suitable base (for example, an inorganic base such as potassium phosphate or cesium fluoride), a metal catalyst (for example, a palladium catalyst such as $Pd(OAc)_2$), and a ligand (for example, a phosphine ligand) according to circumstances.

The metal catalyst and ligand may be added as a preformed complex (for example, a palladium / phosphine complex such as bis (triphenylphosphine) palladium dichloride or a [1,1-bis (diphenylphosphino) ferrocene] palladium dichloride dichloromethane adduct).

Q in the compound of the formula (8) represents a substituted or unsubstituted phenyl group or a substituted or unsubstituted naphthyl group, but the substituent on the phenyl group and the naphthyl group may be appropriately converted even after the reaction.

The compound of the present invention exhibits high herbicidal activity in both methods of soil treatment and foliage treatment under upland farming conditions.

The compound of the present invention is effective against various field weeds and may show selectivity for crops such as maize and wheat.

In addition, the compound of the present invention may exhibit plant growth regulating effects such as growth suppressing effects on useful plants such as crops, ornamental plants and fruit trees.

Further, the compound of the present invention has excellent herbicidal effects on paddy weeds and may show selectivity for rice.

The herbicide of the present invention contains at least one selected from the group consisting of the compound

(I) and a salt of the compound (I) as an active ingredient.

That is, one aspect of the present invention is a herbicide containing at least one selected from the group consisting of the compound (I) and a salt thereof as an active ingredient.

The herbicide of the present invention exhibits high herbicidal activity in both methods of soil treatment and foliage treatment under upland farming conditions.

In addition, the herbicide of the present invention has excellent herbicidal effects on paddy weeds such as Echinochloa spp., Cyperus difforis, Sagittaria trifolia and Schoenoplectiella hotarui, and may show selectivity for rice.

Furthermore, the herbicide of the present invention can also be applied for the control of weeds in places such as orchards, lawns, track ends and vacant sites.

Useful plants for which the herbicide of the present invention can be used include crops such as barley and wheat, cotton, rapeseed, sunflower, maize, rice, soybean, sugar beet, sugar cane and lawn.

Crops may also include trees such as fruit trees, palm trees, coconut trees or other nuts, and also include vines such as grapes, fruit shrubs, fruit plants and vegetables.

Examples of upland field weeds to be controlled include the following weeds.

(A) Monocotyledonous weeds

(1) Weeds of the family Cyperaceae

Weeds of the genus Cyperus such as Cyperus esculentus, Cyperus iria, Cyperus microiria and Cyperus rotundus.

(2) Weeds of the family Poaceae

Weeds of the genus Alopecurus such as Alopecurus aequalis and Alopecurus myosuroides;
Weeds of the genus Apera such as Apera spica-venti;
Weeds of the genus Avena such as Avena sativa;
Weeds of the genus Bromus such as Bromus japonicus and Bromus sterilis;
Weeds of the genus Digitaria such as Digitaria ciliaris and Digitaria sanguinalis;
Weeds of the genus Echinochloa such as Echinochloa crus-galli;
Weeds of the genus Eleusine such as Eleusine indica;
Weeds of the genus Lolium such as Lolium multiflorum Lam.;
Weeds of the genus Panicum such as Panicum dichotomiflorum;
Weeds of the genus Poa such as Poa annua;
Weeds of the genus Setaria such as Setaria faberi, Setaria pumila and Setaria viridis;
Weeds of the genus Sorghum such as Sorghum bicolor; and
Weeds of the genus Urochloa such as Urochloa platyphylla.

(B) Dicotyledonous weeds

(1) Weeds of the family Amaranthaceae

Weeds of the genus Amaranthus such as Amaranthus blitum, Amaranthus palmeri, Amaranthus retroflexus and Amaranthus rudis;
Weeds of the genus Chenopodium such as Chenopodium album;
Weeds of the genus Bassia such as Bassia scoparia.

(2) Weeds of the family Asteraceae

Weeds of the genus Ambrosia such as Ambrosia artemisiifolia and Ambrosia trifida;
Weeds of the genus Conyza such as Conyza canadensis and Conyza sumatrensis;
Weeds of the genus Erigeron such as Erigeron annuus;
Weeds of the genus Matricaria such as Matricaria inodora and Matricaria recutita;
Weeds of the genus Xanthium such as Xanthium occidentale.

(3) Weeds of the family Caryophyllaceae

Weeds of the genus Sagina such as Sagina japonica;
Weeds of the genus Stellaria such as Stellaria media.

(4) Weeds of the family Convolvulaceae

Weeds of the genus Calystegia such as Calystegia japonica;
Weeds of the genus Ipomoea such as Ipomoea coccinea, Ipomoea hederacea, Ipomoea lacunosa and Ipomoea triloba.

(5) Weeds of the family Lamiaceae
Weeds of the genus Lamium such as Lamium album var. barbatum, Lamium amplexicaule and Lamium purpureum.
(6) Weeds of the family Malvaceae

Weeds of the genus Abutilon such as Abutilon theophrasti;
Weeds of the genus Sida such as Sida spinosa.

(7) Weeds of the family Plantaginaceae
Weeds of the genus Veronica such as Veronica persica.
(8) Weeds of the family Polygonaceae

Weeds of the genus Fallopia such as Fallopia convolvulus.
Weeds of the genus Persicaria such as Persicaria lapathifolia and Persicaria longiseta.

(9) Weeds of the family Rubiaceae
Weeds of the genus Galium, such as Galium spurium var. echinospermon.

Examples of paddy weeds to be controlled include the following weeds.

(A) Monocotyledonous weeds

(1) Weeds of the family Alismataceae
Weeds of the genus Sagittaria such as Sagittaria pygmaea Miq. and Sagittaria trifolia.
(2) Weeds of the family Cyperaceae

Weeds of the genus Cyperus such as Cyperus serotinus and Cyperus difforis;
Weeds of the genus Eleocharis such as Eleocharis kuroguwai Ohwi;
Weeds of the genus Schoenoplectiella such as Schoenoplectiella hotarui and Schoenoplectiella juncoides Roxb.
Weeds of the genus Scirpus such as Scirpus maritimus and Scirpus nipponicus.

(3) Weeds of the family Poaceae

Weeds of the genus Echinochloa such as Echinochloa oryzoides and Echinochloa crus-galli;
Weeds of the genus Leersia such as Leersia japonica;
Weeds of the genus Paspalum such as Paspalum distichum.

(4) Weeds of the family Pontederiaceae
Weeds of the genus Monochoria such as Monochoria korsakowii and Monochoria vaginalis var. plantaginea.

(B) Dicotyledonous weeds

(1) Weeds of the family Apiaceae
Weeds of the genus Oenanthe such as Oenanthe javanica.
(2) Weeds of the family Elatinaceae
Weeds of the genus Elatine such as Elatine triandra.
(3) Weeds of the family Linderniaceae
Weeds of the genus Lindernia such as Lindernia dubia subsp. major, Lindernia dubia subsp. dubia and Lindernia procumbens.
(4) Weeds of the family Lythraceae
Weeds of the genus Rotala such as Rotala indica var. uliginosa.

The herbicide of the present invention may consist only of the compound of the present invention, or may be

formulated into a dosage form generally adopted as an agricultural chemical, for example, a wettable powder, a granule, a powder, an emulsion, a water soluble powder, a or a flowable.

A known additive or carrier can be used for formulation.

That is, one aspect of the present invention is a herbicide containing an agrochemically acceptable solid carrier and / or liquid carrier.

For solid dosage forms, vegetable powders such as soy flour and wheat flour, fine mineral powders such as diatomaceous earth, apatite, gypsum, talc, bentonite, pyrophyllite and clay, and solid carriers of organic and inorganic compounds such as sodium benzoate, urea and mirabilite can be used.

For liquid dosage forms, petroleum fractions such as kerosine, xylene and solvent naphtha, and liquid carriers such as cyclohexane, cyclohexanone, dimethylformamide, dimethyl sulfoxide, alcohol, acetone, trichloroethylene, methyl isobutyl ketone, mineral oil, vegetable oil and water can be used.

In formulation, a surfactant can be added as needed. Examples of the surfactant include nonionic surfactants such as alkylphenyl ethers to which polyoxyethylene is added, alkyl ethers to which polyoxyethylene is added, higher fatty acid esters to which polyoxyethylene is added, sorbitan higher fatty acid esters to which polyoxyethylene is added, and tristyrylphenyl ethers to which polyoxyethylene is added, sulfuric acid ester salts of alkylphenyl ethers to which polyoxyethylene is added, alkylnaphthalene sulfonate, polycarboxylate, lignin sulfonate, formaldehyde condensates of alkylnaphthalene sulfonate, and isobutylene-maleic anhydride copolymers.

In the herbicide of the present invention, the concentration of the active ingredient can be appropriately set according to the dosage form. For example, the concentration of the active ingredient in a wettable powder is preferably from 5 to 90% by weight, and more preferably from 10 to 85% by weight. The concentration of the active ingredient in an emulsion is preferably from 3 to 70% by weight, and more preferably from 5 to 60% by weight. The concentration of the active ingredient in a granule is preferably from 0.01 to 50% by weight, and more preferably from 0.05 to 40% by weight.

The wettable powder or emulsion obtained in this manner can be used as a suspension or emulsion by diluting with water to a predetermined concentration, and the granules can be directly sprayed on or mixed with the soil before or after germination of weeds. When the herbicide of the present invention is applied to a farm field, an appropriate amount of 0.1 g or more of the active ingredient per hectare can be applied.

In addition, the herbicide of the present invention can also be used by mixing with a known fungicide, fungicidal active ingredient, insecticide, insecticidal active ingredient, acaricide, acaricidal active ingredient, herbicide, herbicidal active ingredient, plant growth regulator, fertilizer or a phytotoxicity reducing agent (safener).

In particular, it is possible to reduce the amount of drug used by using it in combination with a herbicide. Further, in addition to labor saving, even higher effects can also be expected due to the synergistic action of the mixed drug. In that case, a combination with a plurality of known herbicides is also possible.

That is, one aspect of the present invention is a herbicide containing one or more additional herbicidal active ingredients.

Further, one aspect of the present invention is a herbicide containing one or more additional phytotoxicity reducing agents.

Other herbicidal active ingredients used in the present invention are not particularly limited, and examples thereof include the following.

(a) aryloxyphenoxypropionic acid ester-based ingredients such as clodinafop-propargyl, cyhalofop-butyl, diclofop-methyl, fenoxaprop-P-ethyl, fluazifop-P, fluazifop-P-butyl, haloxyfop-methyl, pyriphenop-sodium, propaquizafop, quizalofop-P-ethyl and metamifop; cyclohexanedione-based ingredients such as alloxydim, butroxydim, clethodim, cycloxydim, profoxydim, sethoxydim, tepraloxydim and tralkoxydim; phenylpyrazolin-based ingredients such as pinoxaden; and other ingredients that are said to exhibit herbicidal efficacies by inhibiting acetyl CoA carboxylase of plants.

(b) sulfonylurea-based ingredients such as amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flupyrsulfuron, foramsulfuron, halosulfuron-methyl, imazosulfuron, iodosulfuron-methyl, mesosulfuron, mesosulfuron-methyl, metsulfuron-methyl, nicosulfuron, oxasulfuron, primisulfuron, prosulfuron, pyrazosulfuron-ethyl, rimsulfuron, sulfometuron-methyl, sulfosulfuron, thifensulfuron-methyl, triasulfuron, tribenuron-methyl, trifloxysulfuron, triflusulfuron-methyl, tritosulfuron, orthosulfamuron, propyrisulfuron, flucetosulfuron, metazosulfuron, methiopyrsulfuron, monosulfuron-methyl, orthosulfuron and iofensulfuron; imidazolinone-based ingredients such as imazapic, imazamethabenz, imazamox-ammonium, imazapyr, imazaquin and imazethapyr; triazolopyrimidine sulfonamide-based ingredients such as cloransulam-methyl, diclosulam, florasulam, flumetsulam, metosulam, penoxsulam, pyroxsulam and metosulfam; pyrimidinyl(thio)benzoate-based ingredients such as bispyribac-sodium, pyribenzoxim, pyriftalid, pyrithiobac-sodium, pyriminobac-methyl and pyrimisulfan; sulfonyl amino carbonyl triazolinone-based ingredients such as flucarbazone, propoxycarbazone and thiencarbazone-methyl; sulfonanilide-based ingredients such as triafamone; and other ingredients that are said to exhibit herbicidal efficacies by inhibiting acetolactate synthase (ALS)

(acetohydroxy acid synthase (AHAS)) of plants.

(c) triazine-based ingredients such as ametryn, atrazine, cyanazine, desmetryne, dimethametryn, prometon, prometryn, propazine-based ingredients (propazine), CAT (simazine), simetryn, terbumeton, terbuthylazine, terbutryne, trietazine, atratone and cybutryne; triazinone-based ingredients such as hexazinone, metamitron and metribuzin; triazolinone-based ingredients such as amicarbazone; uracil-based ingredients such as bromacil, lenacil and terbacil; pyridazinone-based ingredients such as PAC (chloridazon); carbamate-based ingredients such as desmedipham, phenmedipham and swep; urea-based ingredients such as chlorobromuron, chlorotoluron, chloroxuron, dimefuron, DCMU (diuron), ethidimuron, fenuron, fluometuron, isoproturon, isouron, linuron, methabenzthiazuron, metobromuron, metoxuron, monolinuron, neburon, siduron, tebuthiuron, metobenzuron and karbutilate; amide-based ingredients such as DCPA (propanil) and CMMP (pentanochlor); anilide-based ingredients such as cypromid; nitrile-based ingredients such as bromofenoxim, bromoxynil and ioxynil; benzothiadiazinone-based ingredients such as bentazone; phenylpyridazine-based ingredients such as pyridate and pyridafol; and other ingredients that are said to exhibit herbicidal efficacies by inhibiting photosynthesis of plants such as methazole.

(d) bipyridylium-based ingredients such as diquat and paraquat; and other ingredients that are said to become free radicals themselves in plants and generate active oxygen to exhibit fast-acting herbicidal effects.

(e) diphenyl ether-based ingredients such as acifluorfen-sodium, bifenox, chlomethoxynil (chlomethoxyfen), fluoroglycofen, fomesafen, halosafen, lactofen, oxyfluorfen, nitrofen and ethoxyfen-ethyl; phenylpyrazole-based ingredients such as fluazolate and pyraflufen-ethyl; N-phenylphthalimide-based ingredients such as cinidon-ethyl, flumioxazin, flumiclorac-pentyl and chlorphthalim; thiadiazole-based ingredients such as fluthiacet-methyl and thidiazimin; oxaziazole-based ingredients such as oxadiazon and oxadiargyl; triazolinone-based ingredients such as azafenidin, carfentrazone-ethyl, sulfentrazone and bencarbazone; oxazolidinedione-based ingredients such as pentoxazone; pyrimidinedione-based ingredients such as benzfendizone and butafenacil; sulfonylamide-based ingredients such as saflufenacil; pyridazine-based ingredients such as flufenpyr-ethyl; and other ingredients that are said to exhibit herbicidal efficacies by inhibiting chlorophyll biosynthesis in plants and abnormally accumulating photosensitizing peroxide substances in plant bodies, such as pyrachlonil, profluazol, tiafenacil and trifludimoxazin.

(f) pyridazinone-based ingredients such as norflurazon and metflurazon; pyridinecarboxamide-based ingredients such as diflufenican and picolinafen; triketone-based ingredients such as mesotrione, sulcotrione, tefuryltrione, tembotrione, bicyclopyrone and fenquinotrione; isoxazole-based ingredients such as isoxachlortole and isoxaflutole; pyrazole-based ingredients such as benzofenap, pyrazolynate, pyrazoxyfen, topramezone, pyrasulfotole and tolpyralate; triazole-based ingredients such as ATA (amitrol); isooxazolidinone-based ingredients such as clomazone; diphenyl ether-based ingredients such as aclonifen; and other ingredients that are said to exhibit herbicidal efficacies by inhibiting the biosynthesis of plant pigments such as carotenoids characterized by a bleaching action such as beflubutamid, fluridone, flurochloridone, flurtamone, benzobicyclone, methoxyphenone and ketospiradox.

(g) glycine-based ingredients such as glyphosate, glyphosate-ammonium, glyphosate-isopropylamine and glyphosate trimesium (sulfosate); and other ingredients inhibiting EPSP synthase

(h) phosphinic acid-based ingredients inhibiting glutamine synthetase such as glufosinate, glufosinate-ammonium, and bialaphos (bilanafos), and

other ingredients that are said to exhibit herbicidal efficacies by inhibiting the amino acid biosynthesis of plants.

(i) carbamate-based ingredients such as asulam; and other ingredients inhibiting DHP (dihydropteroate) synthase

(j) dinitroaniline-based ingredients such as bethrodine (benfluralin), butralin, dinitramine, ethalfluralin, oryzalin, pendimethalin, trifluralin, nitralin and prodiamine; phosphoroamidate-based ingredients such as amiprofos-methyl and butamifos; pyridine-based ingredients such as dithiopyr and thiazopyr; benzamide-based ingredients such as propyzamide and tebutam; benzoic acid-based ingredients such as chlorthal and TCTP (chlorthal-dimethyl); carbamate-based ingredients such as IPC (chlorpropham), propham, carbetamide and barban; arylalanine-based ingredients such as flamprop-M and flamprop-M-isopropyl; chloroacetamide-based ingredients such as acetochlor, alachlor, butachlor, dimethachlor, dimethenamid, dimethenamid-P, metazachlor, metolachlor, S-metolachlor, pethoxamid, pretilachlor, propachlor, propisochlor and thenylchlor; acetamide-based ingredients such as diphenamid, napropamide and naproanilide; oxyacetamide-based ingredients such as flufenacet and mefenacet; tetrazolinone-based ingredients such as fentrazamide; and other ingredients that are said to exhibit herbicidal efficacies by inhibiting the microtubule polymerization, microtubule formation and cell division of plants or by inhibiting the biosynthesis of very long chain fatty acids (VLCFA), such as anilofos, indanofan, cafenstrole, piperophos, methiozolin, fenoxasulfone, pyroxasulfone and ipfencarbazone.

(k) nitrile-based ingredients such as DBN (dichlobenil) and DCBN (chlorthiamid); benzamide-based ingredients such as isoxaben; triazolocarboxamide-based ingredients such as flupoxam; quinoline carboxylic acid-based ingredients such as quinclorac; and other ingredients that are said to exhibit herbicidal efficacies by inhibiting the cell wall (cellulose) synthesis such as triaziflam and indaziflam.

(l) dinitrophenol-based ingredients such as DNOC, DNBP (dinoseb) and dinoterb; and other ingredients that are said to exhibit herbicidal efficacies by uncoupling (membrane disruption).

(m) thiocarbamate-based ingredients such as butylate, hexylthiocarbam (cycloate), dimepiperate, EPTC, esprocarb, molinate, orbencarb, pebulate, prosulfocarb, benthiocarb (thiobencarb), tiocarbazil, triallate, vernolate and diallate; phosphorodithioate-based ingredients such as SAP (bensulide); benzofuran-based ingredients such as benfuresate and ethofumesate; chlorocarbonic acid-based ingredients such as TCA, DPA (dalapon) and tetrapion (flupropanate); and other ingredients that are said to exhibit herbicidal efficacies by inhibiting the lipid biosynthesis of plants.

(n) phenoxycarboxylic acid-based ingredients such as clomeprop, 2,4-PA (2,4-D), 2,4-DB, dichlorprop, MCPA, MCPB and MCPP (mecoprop); benzoic acid-based ingredients such as chloramben, MDBA (dicamba) and TCBA (2,3,6-TBA); pyridinecarboxylic acid-based ingredients such as clopyralid, aminopyralid, fluroxypyr, picloram, triclopyr and halauxifen; quinoline carboxylic acid-based ingredients such as quinclorac and quinmerac; phthalamate semicarbazone-based ingredients such as NPA (naptalam) and diflufenzopyr; and other ingredients that are said to exhibit herbicidal efficacies by disturbing the hormone action of plants such as benazolin, diflufenzopyr, fluroxypyr, chlorflurenol, aminocyclopyrachlor, and DAS534.

(o) arylaminopropionic acid-based ingredients such as flamprop-isopropyl; pyrazolium-based ingredients such as difenzoquat; organic arsenic-based ingredients such as DSMA and MSMA; and other herbicides such as bromobutide, chlorflurenol, cinmethylin, cumyluron, dazomet, daimuron, methyl-dymron, etobenzanid, fosamine, oxaziclomefone, oleic acid, pelargonic acid, pyributicarb, endothall, sodium chlorate, metam, quinoclamine, cyclopyrimorate, tridiphane and clacyfos.

Examples of the phytotoxicity reducing agent (safener) that can be used in the present invention include benoxacor, cloquintocet, cloquintocet-mexyl, cyometrinil, cyprosulfamide, dichlormid, dicyclonon, dietholate, fenchlorazole, fenchlorazole-ethyl, fenclorim, flurazole, fluxofenim, furilazole, isoxadifen, isoxadifen-ethyl, mefenpyr, mefenpyr-diethyl, mephenate, naphthalic anhydride and oxabetrinil.

EXAMPLES

[Formulation Examples]

Although some formulation examples relating to the herbicide of the present invention are shown, the compounds (active ingredients), additives and addition ratios of the present invention are not limited only to these examples and can be changed in a wide range. The term "part" in the formulation examples indicates "part by weight".

(Formulation Example 1) Wettable powder

| | |
|---|---|
| Compound of the present invention | 20 parts |
| White carbon | 20 parts |
| Diatomaceous earth | 52 parts |
| Sodium alkyl sulfate | 8 parts |

The above components are mixed uniformly and finely pulverized to obtain a wettable powder containing 20% of an active ingredient.

(Formulation Example 2) Emulsion

| | |
|---|---|
| Compound of the present invention | 20 parts |
| Xylene | 55 parts |
| Dimethylformamide | 15 parts |
| Polyoxyethylene phenyl ether | 10 parts |

The above components are mixed and dissolved to obtain an emulsion containing 20% of an active ingredient.

(Formulation Example 3) Granule

| | |
|---|---|
| Compound of the present invention | 5 parts |
| Talc | 40 parts |
| Clay | 38 parts |
| Bentonite | 10 parts |
| Sodium alkyl sulfate | 7 parts |

The above components are uniformly mixed and finely pulverized, and then granulated into a granular form having a diameter of 0.5 to 1.0 mm to obtain a granule containing 5% of an active ingredient.

Next, synthesis examples will be shown. However, the present invention is not limited to the following examples.

(Example 1)

Production of 1-methoxy-3-methyl-6-(2-(methylsulfonyl)-4-(trifluoromethyl)phenyl)-7-oxa-3,4-diazabicyclo [4.1.0] hept-4-en-2-one [1-methoxy-3-methyl-6-(2-(methylsulfonyl)-4-(trifluoromethyl) phenyl)-7-oxa-3,4-diazabicyclo [4.1.0] hept-4-en-2-one] (Compound No. A-1)

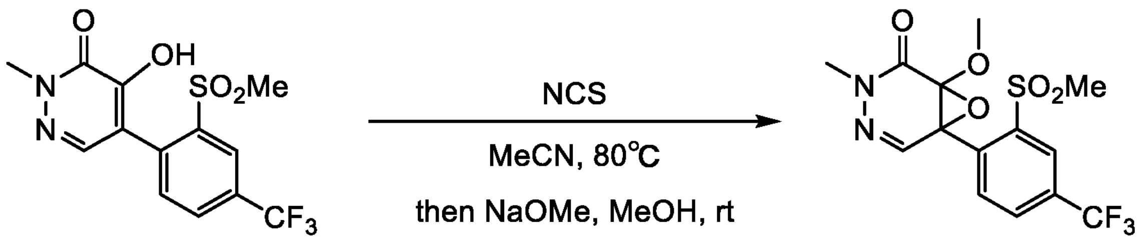


4-hydroxy-2-methyl-5-(2-(methylsulfonyl)-4-(trifluoromethyl)phenyl) pyridazin-3(2H)-one (0.09 g) was dissolved in acetonitrile (2.5 mL), and the resulting mixture was stirred at room temperature. N-chlorosuccinimide (0.10 g) was added thereto, and the resulting mixture was stirred at 80°C for 3 hours, and then cooled to room temperature. Then, methanol (2.5 mL) and sodium methoxide (0.05 g) were added thereto, and the resulting mixture was stirred at the same temperature for 1 hour.

The reaction solution was concentrated under reduced pressure. The obtained concentrate was purified by silica gel column chromatography to obtain 0.05 g of a target compound.

(Example 2)

Synthesis of 1-methoxy-3-methyl-6-(1,1-dioxido-8-(trifluoromethyl)thiochroman-5-yl)-7-oxa-3,4-diazabicyclo [4.1.0] hept-4-en-2-one [1-methoxy-3-methyl-6-(1,1-dioxido-8-(trifluoromethyl) thiochroman-5-yl)-7-oxa-3,4-diazabicyclo [4.1.0] hept-4-en-2-one]: (Compound No. B-1)

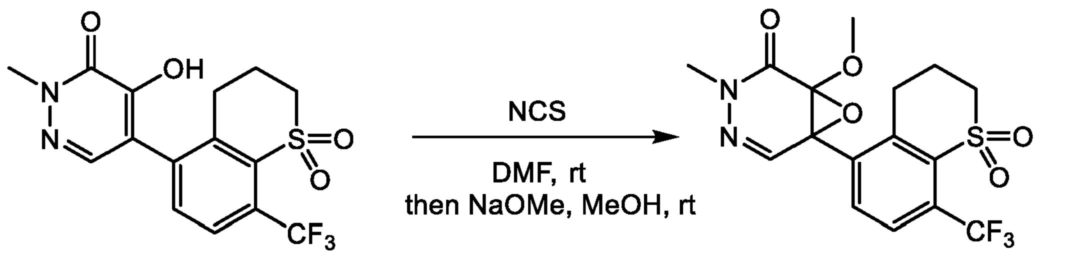

5-(1,1-dioxide-8-(trifluoromethyl)thiochroman-5-yl)-4-hydroxy-2-methylpyridazin-3(2H)-one (0.37 g) was dissolved in N,N-dimethylformamide (2 mL) and the resulting mixture was stirred at room temperature. N-chlorosuccinimide (0.10 g) was added thereto, and the resulting mixture was stirred at room temperature for 2 hours. Then, methanol (10 mL) and sodium methoxide (0.22 g) were added thereto, and the resulting mixture was stirred at the same temperature for 1 hour.

The reaction solution was concentrated under reduced pressure, and the obtained residue was purified by silica gel column chromatography to obtain 0.23 g of a target product.

(Reference Example 1)

Synthesis of 5-(1,1-dioxide-8-(trifluoromethyl)thiochroman-5-yl)-4-hydroxy-2-methylpyridazin-3(2H)-one

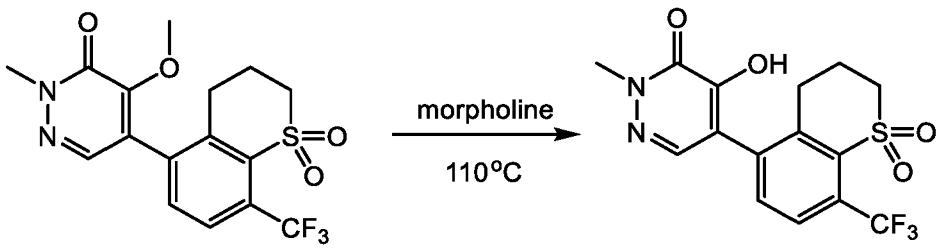


5-(1,1-dioxide-8-(trifluoromethyl)thiochroman-5-yl)-4-methoxy-2-methylpyridazin-3(2H)-one (0.39 g) was dissolved in morpholine (2 mL), and then the resulting mixture was heated under reflux at 110°C for 1 hour.

The reaction solution was poured into hydrochloric acid and then extracted with ethyl acetate. The obtained organic layer was washed with saturated brine, dried over anhydrous magnesium sulfate, and filtered. The filtrate was concentrated under reduced pressure to obtain 0.31 g of a target product.

(Reference Example 2)

Synthesis of 5-(1,1-dioxide-8-(trifluoromethyl)thiochroman-5-yl)-4-methoxy-2-methylpyridazin-3(2H)-one

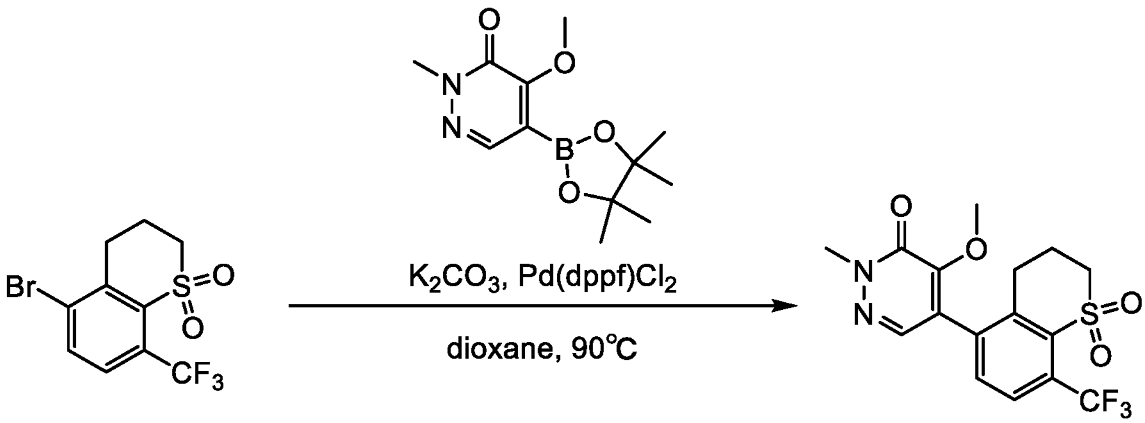


5-Bromo-8-(trifluoromethyl)thiochroman 1,1-dioxide (0.36 g) was dissolved in dioxane (10 mL), and the resulting mixture was stirred at room temperature. 4-methoxy-2-methyl-5-(4,4,5,5-tetramethyl-1,3,2-dioxaborolan-2-yl)pyridazin-3(2H)-one (0.27 g), potassium carbonate (0.41 g) and [1,1'-bis(diphenylphosphino) ferrocene] palladium (II) dichloride-dichloromethane adduct (0.04 g) were added thereto sequentially, and the resulting mixture was stirred at 90°C overnight.

The obtained liquid was filtered. The filtrate was concentrated under reduced pressure. The obtained concentrate was purified by silica gel column chromatography to obtain 0.22 g of a target compound.

The NMR data of the obtained compound are shown.

$^1$H-NMR (400 MHz, $CDCl_3$): δ2.40-2.48 (m, 2H), 2.72-2.80 (m, 1H), 2.86-2.95 (m, 1H), 3.40 (t, 2H), 3.83 (s,

3H), 4.12 (s, 3H), 7.41 (d, 1H), 7.44 (s, 1H), 7.84 (d, 1H).

(Reference Example 3)

Synthesis of 5-bromo-8-(trifluoromethyl) thiochroman 1,1-dioxide

(Step 1)

Synthesis of 3-(6-bromo-2-trifluoro-3-(trifluoromethyl)phenyl) propionic acid

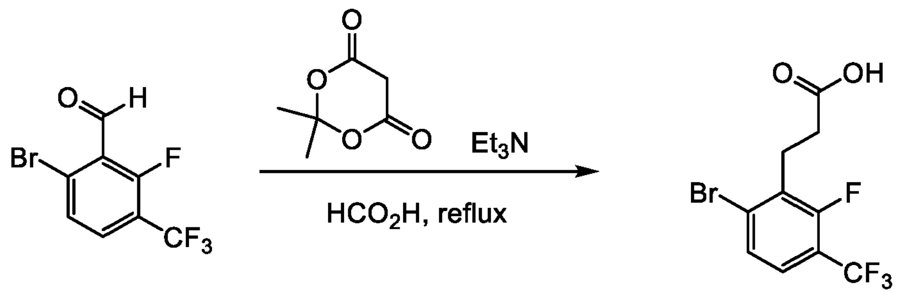


Formic acid (58.2 g), triethylamine (18.3 g), 6-bromo-2-fluoro-3-(trifluoromethyl) benzaldehyde (48.7 g) and Meldrum's acid (26.0 g) were added sequentially at 0°C into a 500 mL four-necked flask. Thereafter, the resulting mixture was heated under reflux for 4 hours.

Hydrochloric acid was added to the obtained liquid, and the precipitated solid substance was separated by filtration. The obtained solid substance was dried to obtain 53.8 g of a target compound.

(Step 2)

Synthesis of 3-(6-bromo-2-fluoro-3-(trifluoromethyl)phenyl) propan-1-ol

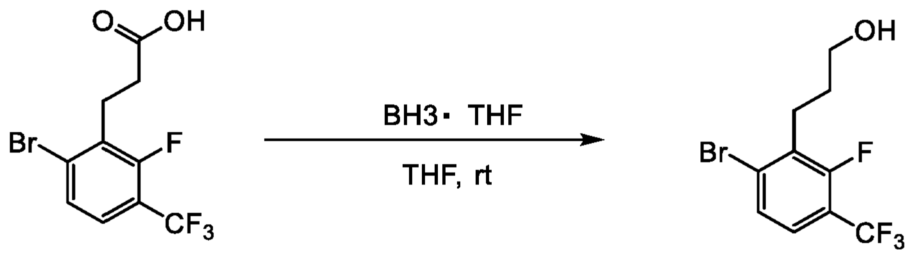


3-(6-Bromo-2-trifluoro-3-(trifluoromethyl)phenyl) propionic acid (30 g) was dissolved in tetrahydrofuran (191 mL), and the resulting mixture was stirred at room temperature. A borane-tetrahydrofuran complex (0.9M, 127 mL) was added thereto, and the resulting mixture was stirred at 0°C for 1 hour.

The obtained liquid was poured into hydrochloric acid and then extracted with ethyl acetate. The obtained organic layer was washed with saturated brine, dried over anhydrous magnesium sulfate, and filtered. The filtrate was concentrated under reduced pressure. The obtained concentrate was purified by silica gel column chromatography to obtain 28.9 g of a target compound.

(Step 3)

Synthesis of 1-bromo-2-(3-chloropropyl)-3-fluoro-4-(trifluoromethyl) benzene

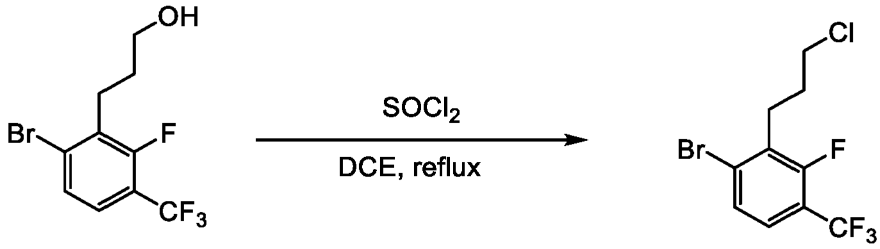


3-(6-Bromo-2-fluoro-3-(trifluoromethyl)phenyl) propan-1-ol (15.3 g) was dissolved in dichloroethane (102 mL), and the resulting mixture was stirred at room temperature. Thionyl chloride (9.1 g) and N,N-dimethylformamide (0.2 g) were added thereto, and the resulting mixture was heated under reflux for 2 hours.

The obtained liquid was concentrated under reduced pressure. The obtained liquid was concentrated under reduced pressure. The obtained concentrate was purified by silica gel column chromatography to obtain 16.4 g of a target compound.

(Step 4)

Synthesis of 5-bromo-8-(trifluoromethyl) thiochroman

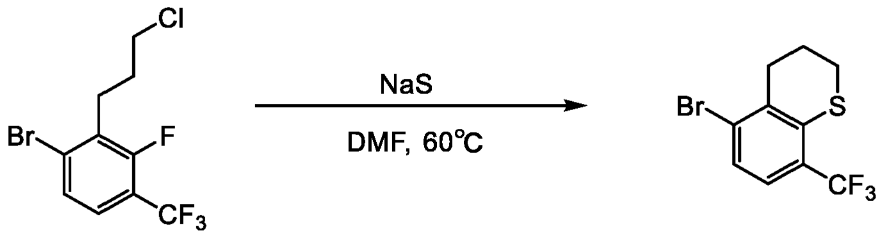


1-Bromo-2-(3-chloropropyl)-3-fluoro-4-(trifluoromethyl) benzene (14.4 g) was dissolved in N,N-dimethylformamide (158 mL), and the resulting mixture was stirred at room temperature. Sodium sulfide (4.2 g) was added thereto, and the resulting mixture was heated under reflux at 60°C overnight.

The obtained liquid was poured into water and then extracted with ethyl acetate. The obtained organic layer was washed with saturated brine, dried over anhydrous magnesium sulfate, and filtered. The filtrate was concentrated under reduced pressure. The obtained concentrate was purified by silica gel column chromatography to obtain 6.8 g of a target compound.

(Step 5)

Synthesis of 5-bromo-8-(trifluoromethyl) thiochroman 1,1-dioxide

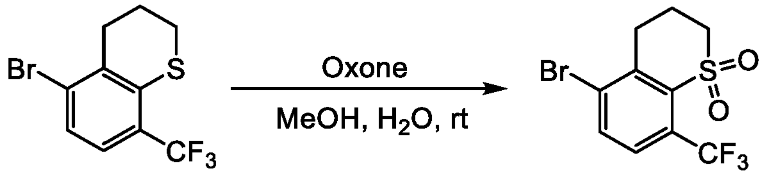


5-Bromo-8-(trifluoromethyl) thiochroman (2.0 g) was dissolved in 27 mL of methanol and 7 mL of water, and the resulting mixture was stirred at room temperature. Oxone (8.3 g) was added thereto, and the resulting mixture was stirred at room temperature for 48 hours.

The obtained liquid was filtered. The filtrate was concentrated under reduced pressure. Water was poured thereinto and then extracted with ethyl acetate. The obtained organic layer was washed with saturated brine, dried over anhydrous magnesium sulfate, and filtered. The filtrate was concentrated under reduced pressure. The obtained concentrate was purified by silica gel column chromatography to obtain 2.0 g of a target compound.

(Reference Example 4)

Synthesis of 5-(1,1-dioxide-8-(trifluoromethyl)thiochroman-5-yl)-4-methoxy-2-methylpyridazin-3(2H)-one

(Step 1)

Synthesis of 5-(8-(trifluoromethyl) thiochroman-5-yl)-4-methoxy-2-methylpyridazin-3(2H)-one

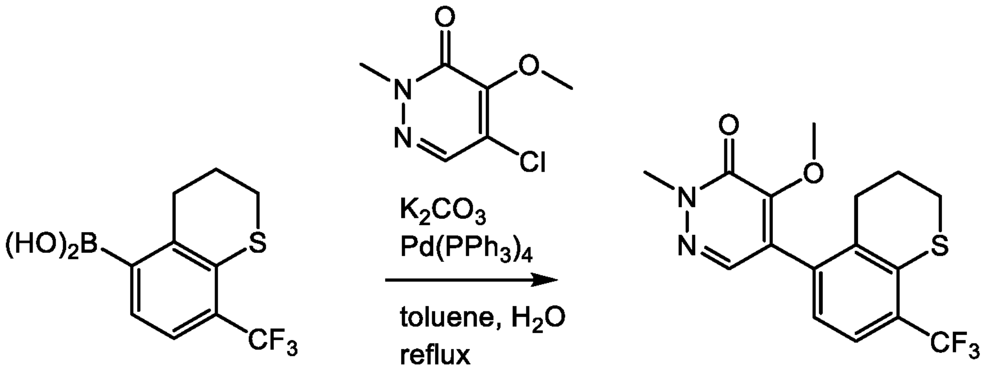


(8-(Trifluoromethyl) thiochroman-5-yl) boronic acid (9.6 g) was dissolved in toluene (200 mL) and water (50 mL), and the resulting mixture was stirred at room temperature. 5-chloro-4-methoxy-2-methylpyridazin-3(2H)-one (13.1 g), potassium carbonate (17.3 g) and tetrakis (triphenylphosphine) palladium (0) (4.0 g) were sequentially added thereto, and the resulting mixture was heated under reflux overnight.

Water was poured thereinto and then extracted with ethyl acetate. The obtained organic layer was washed with saturated brine, dried over anhydrous magnesium sulfate, and filtered. The filtrate was concentrated under reduced pressure. The obtained concentrate was purified by silica gel column chromatography to obtain 16.8 g of a target compound.

(Step 2)

Synthesis of 5-(1,1-dioxide-8-(trifluoromethyl) thiochroman-5-yl)-4-methoxy-2-methylpyridazin-3(2H)-one

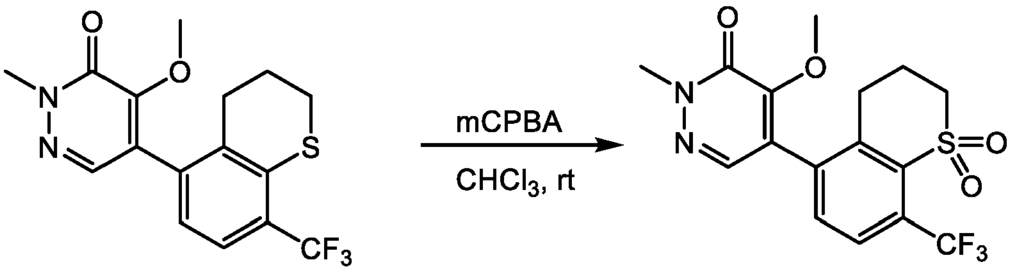


4-Methoxy-2-methyl-5-(8-(trifluoromethyl) thiochroman-5-yl) pyridazin-3(2H)-one (1.5 g) was dissolved in chloroform (16 mL), and the resulting mixture was stirred at 0°C. Meta-chloroperoxybenzoic acid (2.4 g) was added to this solution, and the resulting mixture was stirred at room temperature for 2 hours.

Water, an aqueous sodium thioacetate solution, and a saturated aqueous sodium bicarbonate solution were sequentially added thereto, and the resulting mixture was concentrated under reduced pressure. The concentrated liquid was extracted with ethyl acetate. The obtained organic layer was washed with a saturated aqueous sodium bicarbonate solution and saturated brine, dried over anhydrous magnesium sulfate, and filtered. The filtrate was concentrated under reduced pressure. The obtained crystals were dried to obtain 1.6 g of a target product.

(Reference Example 5)

Synthesis of (8-(trifluoromethyl) thiochroman-5-yl) boronic acid

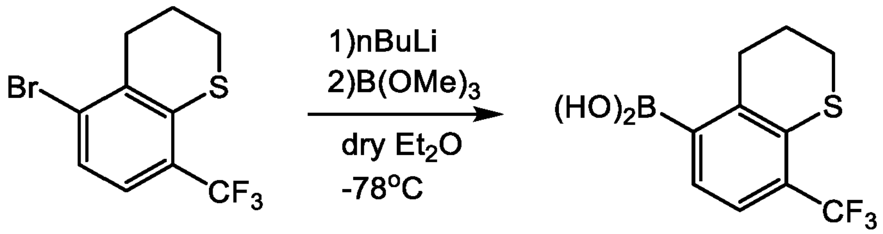


5-Bromo-8-(trifluoromethyl) thiochroman (4.5 g) was dissolved in ether (30 mL), and the resulting mixture was stirred at -78°C. An n-butyllithium-hexane solution (2.8 M, 5.9 mL) was added to this solution, and the resulting mixture was stirred at the same temperature for 1 hour. Trimethylborate (2.0 mL) was added, and the temperature was gradually raised to room temperature.

Hydrochloric acid was poured thereinto and then extracted with ethyl acetate. The obtained organic layer was washed with saturated brine, dried over anhydrous magnesium sulfate, and filtered. The filtrate was concentrated under reduced pressure. The precipitated solid substance was separated by filtration, and the obtained solid substance was dried to obtain 2.8 g of a target compound.

Table 1 shows an example of the compound of the present invention produced by the same method as in the above synthesis example. Table 1 shows the substituents in the compound represented by the formula (I-2). At the same time, the melting point is also shown as a physical property value. Me represents a methyl group, Et represents an ethyl group, $^{n}$Pr represents an n-propyl group, $^{i}$Pr represents an i-propyl group, $^{c}$Pr represents a cyclopropyl group, $^{t}$Bu represents a t-butyl group, $^{n}$Hex represents an n-hexyl group and Ph represents a phenyl group. Hereinafter, they have the same meanings in Tables 2 and 3.

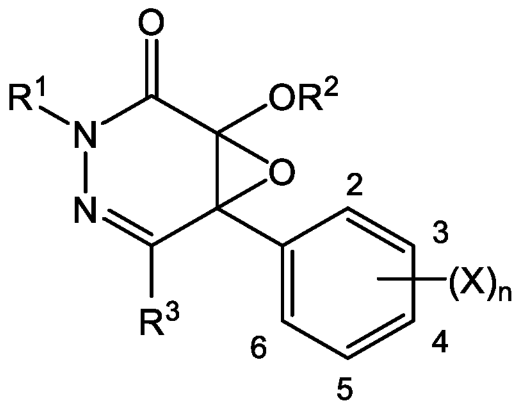

(I-2)

Table 1

| Compound Number | $R^1$ | $R^2$ | $R^3$ | (X)n | Physical property value |
|---|---|---|---|---|---|
| A-1 | Me | Me | H | 2-$SO_2$Me, 4-$CF_3$ | m.p. 128°C |
| A-2 | Me | Me | Me | 2-$SO_2$Me, 4-$CF_3$ | m.p. 143-144°C |
| A-3 | Me | Me | H | 2-Cl, 3-$SO_2$Me, 4-$CF_3$ | m.p. 171-173°C |
| A-4 | Me | Me | H | 2-Cl, 3-$CH_2OCH_2$$^{c}$Pr, 4-$SO_2$Me | m.p. 69-70°C |
| A-5 | Me | Me | H | 2-Cl, 3-Me, 4-$SO_2$Me | m.p. 196-197°C |

(continued)

| Compound Number | $R^1$ | $R^2$ | $R^3$ | (X)n | Physical property value |
|---|---|---|---|---|---|
| A-6 | Me | Me | H | 2-Cl, 4-$SO_2$Me | m.p. 188-189°C |
| A-7 | Me | Me | H | 2-$NO_2$, 4-$SO_2$Me | m.p. 107-108°C |
| A-8 | Me | Me | H | 2-Cl, 4-$CF_3$ | m.p. 83-85°C |
| A-9 | Me | Me | H | 2,4-$Cl_2$ | m.p. 117-118°C |
| A-10 | Me | Me | H | 2-$SO_2$Me | * |
| A-11 | Me | Me | H | 2-Cl, 3-N(OMe)$CO_2{}^t$Bu, 4-$CF_3$ | * |
| A-12 | Me | Me | H | 2-Cl, 3-$OCH_2$Ph, 4-$CF_3$ | m.p. 125-127°C |
| A-13 | Me | Me | H | 2-Cl, 3-$CH_2OCH_2CH_2$OMe, 4-$SO_2$Me | m.p. 93-94°C |
| A-14 | Me | Me | H | 2-CN | m.p. 100-105°C |
| A-15 | Me | Me | H | 2-Cl, 3-CON$(Me)_2$, 4-$SO_2$Me | * |
| A-16 | Me | Me | H | 2-Cl, 3-(4,5-dihydroisoxazol-3-yl), 4-$SO_2$Me | m.p. 110-111°C |
| A-17 | Me | Me | H | 2-Cl, 3-$OCH_2CH_2$OMe, 4-$SO_2$Me | * |
| A-18 | Me | Me | H | 2-$SO_2$Me, 4-$^c$Pr | m.p. 71-75°C |
| A-19 | Me | Me | H | 2-$SO_2$Me, 4-$CH_2OCH_2CH_2$OMe | m.p. 130-131°C |
| A-20 | Me | Me | H | 2-$SO_2$Me, 4-$CH_2OCH_2CH_2CF_3$ | * |
| A-21 | Me | Me | H | 2-Me, 3-NHCO$^c$Pr, 4-$CF_3$ | m.p. 153-154°C |
| A-22 | Me | Me | H | 2-$SO_2$Me, 4-OMe | m.p. 109-112°C |
| A-23 | Me | Me | H | 2-Cl, 3-$CH_2$ (1H-1,2,4-triazol-1-yl), 4-$SO_2$Me | m.p. 78-80°C |
| A-24 | Me | Me | H | 2-Cl, 3- (1H-pyrazol-1-yl), 4-$SO_2$Me | * |
| A-25 | Me | Me | H | 2-Cl, 3-$CH_2$ (morpholino), 4-$SO_2$Me | * |
| A-26 | Me | Me | H | 2-$SO_2$Me, 4-Ph | * |
| A-27 | Me | Me | H | 2-$SO_2$Me, 4-(morpholine-4-carbonyl) | m.p. 108-112°C |
| A-28 | Me | Me | H | 2-$SO_2$Me, 4-$CH_2$OMe | * |
| A-29 | Me | Me | H | 2-$SO_2$Me, 4-$CO_2$Me | m.p. 162-164°C |
| A-30 | Me | Me | H | 2-Cl, 3-(morpholine-4-carboxamido), 4-$SO_2$Me | m.p. 113-114°C |
| A-31 | Me | Me | H | 2-Me, 3-NHCO (morpholine-4-carbonyl), 4-$CF_3$ | * |
| A-32 | Me | Me | H | 2-Me, 3-(morpholine-4-carboxamido), 4-$CF_3$ | * |
| A-33 | Me | Me | H | 2-Cl, 3-(2-oxopyrrolidin-1-yl), 4-$SO_2$Me | * |
| A-34 | Me | Me | H | 2-OMe, 4-$SO_2$Me | m.p. 177-178°C |
| A-35 | Me | Me | H | 2-Me, 3-$CH_2OCH_2CF_3$, 4-$SO_2$Me | * |

(continued)

| Compound Number | $R^1$ | $R^2$ | $R^3$ | (X)n | Physical property value |
|---|---|---|---|---|---|
| A-36 | Me | Me | H | 2-$SO_2CF_3$ | m.p. 104-105°C |
| A-37 | Me | Me | H | 2-Cl, 3-$OCH_2CF_3$, 4-$SO_2Me$ | m.p. 192-193°C |
| A-38 | Me | Me | H | 2-Cl, 3-$OCH_2CONH$ Me, 4-$SO_2Me$ | * |
| A-39 | Me | Me | H | 2-$SO_2Me$, 4-$CF_3$, 5-Cl | m.p. 170-171°C |
| A-40 | Me | Me | H | 2,4-$Cl_2$, 3-NHCO (morpholine-4-carbonyl) | * |
| A-41 | Me | Me | H | 2,4-$(Me)_2$, 3-C(Me)=N-OMe | * |
| A-42 | Me | Me | H | 2-$SO_2Me$, 3-Cl, 4-$CF_3$ | * |
| A-43 | Me | Me | H | 2-Cl, 3-OCON $(Me)_2$, 4-$SO_2Me$ | * |
| A-44 | Me | Me | H | 2-$SO_2Me$, 4-$NO_2$ | m.p. 139-140°C |
| A-45 | Me | Me | H | 2-Cl, 3-$CH_2ON=C(Me)_2$, 4-$SO_2Me$ | * |
| A-46 | Me | Me | cPr | 2-$SO_2Me$, 4-$CF_3$ | m.p. 154-156°C |
| A-47 | $CF_3CH_2$ | Me | H | 2-$SO_2Me$, 4-$CF_3$ | * |
| A-48 | Me | Me | H | 2-Me, 3-(morpholine-4-carboxamido), 4-Cl | 159-162°C |
| A-49 | Me | Me | H | 2-Me, 3-(4-Cl-Ph), 4-$SO_2Me$ | * |
| A-50 | Me | $^n$Hex | H | 2-$SO_2Me$, 4-$CF_3$ | * |
| A-51 | Me | Me | H | 2-$CF_3$, 4-CN | m.p. 156-161°C |
| A-52 | Me | Me | H | 2-Cl, 4-(1H-1,2,4-triazol-1-yl) | * |
| A-53 | Me | Me | H | 2-F, 6-$SO_2Me$ | * |
| A-54 | Me | Me | $^n$PrO | 2-$SO_2Me$, 4-$CF_3$ | * |
| A-55 | Me | Me | H | 2-Me, 6-$SO_2Me$ | m.p. 139-140°C |
| A-56 | Me | Me | H | 2-Cl, 4- (4-Cl-1H-pyrazol-1-yl) | * |
| A-57 | Me | Me | H | 2-$CF_3$, 4-$SO_2Me$ | m.p. 147°C |
| A-58 | Me | $^i$Pr | H | 2-$SO_2Me$, 4-$CF_3$ | m.p. 159-163°C |
| A-59 | Me | Me | H | 2-Me, 3-$SO_2Me$, 4-$CF_3$ | m.p. 180-181°C |
| A-60 | Me | Et | H | 2-$SO_2Me$, 4-$CF_3$ | * |
| A-61 | $^c$Pr$CH_2$ | Me | H | 2-$SO_2Me$, 4-$CF_3$ | * |
| A-62 | Me | $CF_3CH_2$ | H | 2-$SO_2Me$, 4-$CF_3$ | * |
| A-63 | $MeOCH_2$ | Et | H | 2-$SO_2Me$, 4-$CF_3$ | * |
| A-64 | $MeOCH_2$ | Me | H | 2-$SO_2Me$, 4-$CF_3$ | * |
| A-65 | Me | Me | 4-FPh | 2-$SO_2Me$, 4-$CF_3$ | m.p. 144-145°C |
| A-66 | Me | Me | H | 2-Cl, 4-$SO_2Ph$ | m.p. 179-183°C |
| A-67 | Me | Me | H | 2,4-$Cl_2$, 3-$NHSO_2Me$ | m.p. 166-167°C |
| A-68 | Me | Me | H | 2-(morpholino), 3-CN, 4-$CF_3$ | m.p. 124-128°C |

(continued)

| Compound Number | $R^1$ | $R^2$ | $R^3$ | (X)n | Physical property value |
|---|---|---|---|---|---|
| A-69 | Me | Me | H | 2-$CH_2CH_2SO_2$Me, 4-$CF_3$ | * |
| A-70 | Me | Me | H | 2-CH=CH-$SO_2$Me, 4-$CF_3$ | * |
| A-71 | Me | Me | H | 2-Cl, 3-$CH_2SO_2$Et, 4-$SO_2$Me | * |
| A-72 | Me | Me | H | 2-Cl, 3-$^c$Pr, 4-$CF_3$ | m.p. 105-106°C |
| A-73 | Me | Me | H | 2-$SO_2$Me, 4-NHCOMe | m.p. 170-187°C |
| A-74 | Me | Me | H | 2-$SO_2$(morpholino), 4-$CF_3$ | * |
| A-75 | Me | Me | H | 2-$SO_2$Me, 4-$OCF_3$ | * |
| A-76 | Me | Me | H | 2-Cl, 4-$SO_2$(morpholino) | m.p. 153-157°C |
| A-77 | Me | Me | H | 2-$SO_2$Me, 4-C(=N-OMe) Me | m.p. 138-140°C |

Table 2 further shows an example of the compound of the present invention produced by the same method.

Table 2

| Compound Number | Structural formula | Physical property value |
|---|---|---|
| B-1 | 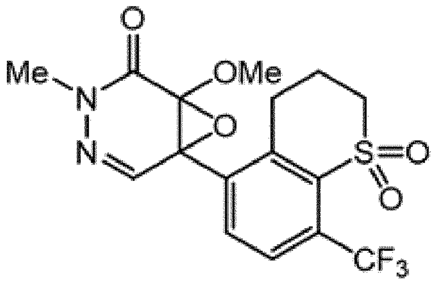 | m.p. 199-200°C |
| B-2 | 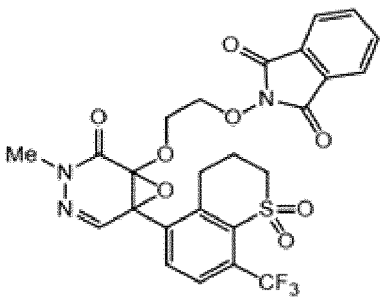 | * |
| B-3 | 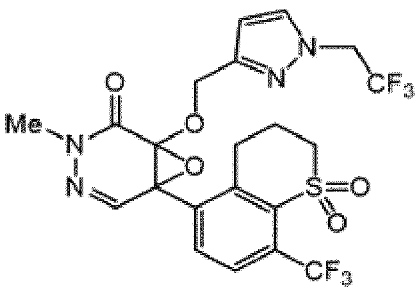 | m.p. 112-117°C |

(continued)

| Compound Number | Structural formula | Physical property value |
|---|---|---|
| B-4 | 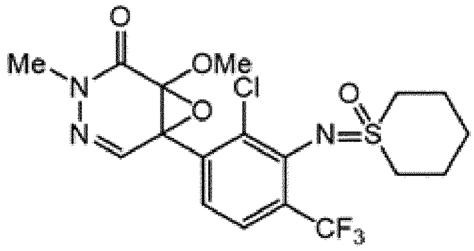 | m.p. 158-161°C |
| B-5 | 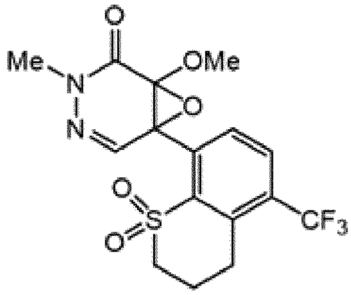 | m.p. 132-134°C |
| B-6 | 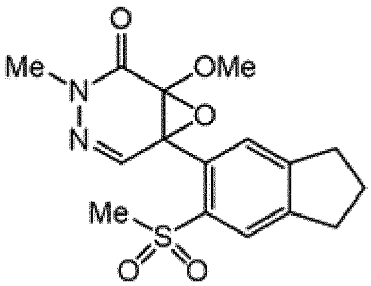 | m.p. 150-153°C |
| B-7 | 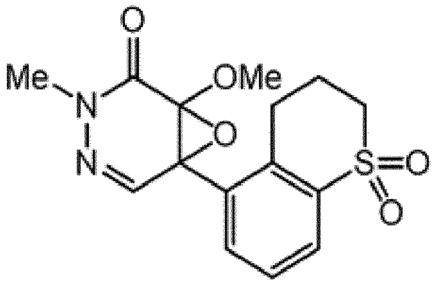 | m.p. 179-181°C |
| B-8 | 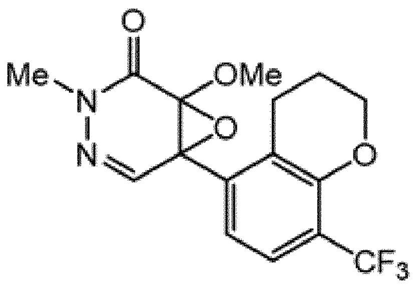 | m.p. 153-155°C |

(continued)

| Compound Number | Structural formula | Physical property value |
|---|---|---|
| B-9 | 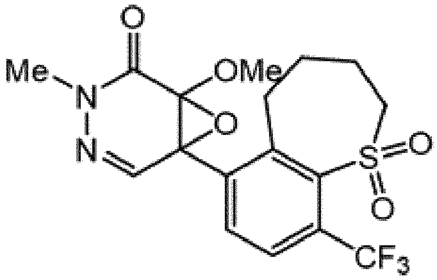 | * |
| B-10 | 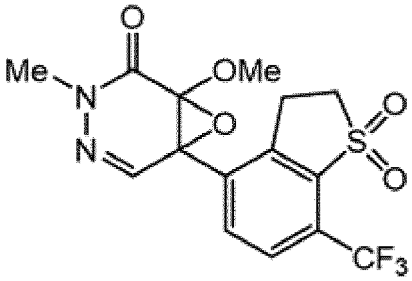 | m.p. 172-177°C |
| B-11 | 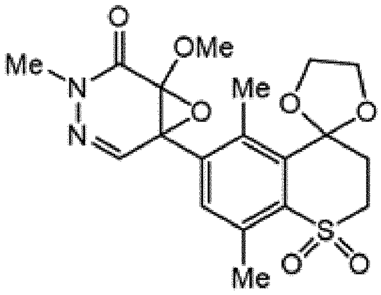 | * |
| B-12 | 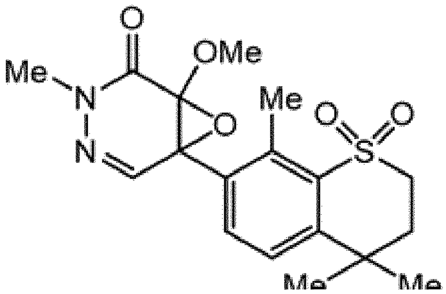 | * |
| B-13 | 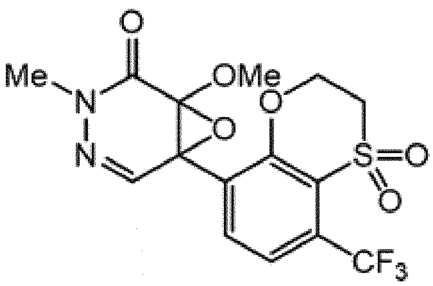 | * |

(continued)

| Compound Number | Structural formula | Physical property value |
|---|---|---|
| B-14 | 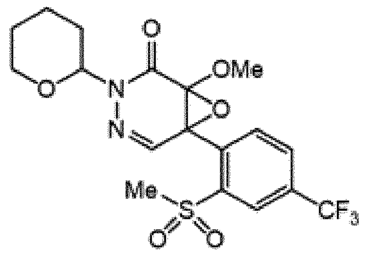 | m.p. 65-66°C |
| B-15 | 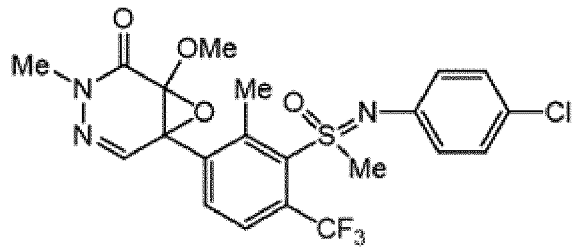 | * |
| B-16 | 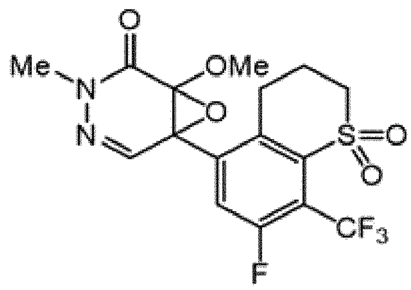 | m.p. 205-206°C |

Table 3 further shows an example of the compound of the present invention produced by the same method. Table 3 shows substituents in the compound represented by the formula (I-4).

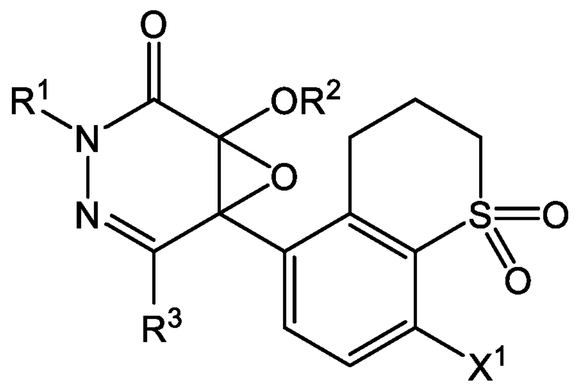

(I-4)

Table 3

| Compound Number | $R^1$ | $R^2$ | R3 | $X^1$ | Physical property value |
|---|---|---|---|---|---|
| C-1 | Me | $CH_2CH_2O$(4-BrPh) | H | $CF_3$ | m.p. 120-124°C |
| C-2 | Me | $CH_2CH_2OCH_2CH_2OMe$ | H | $CF_3$ | m.p. 108-113°C |

(continued)

| Compound Number | $R^1$ | $R^2$ | R3 | $X^1$ | Physical property value |
|---|---|---|---|---|---|
| C-3 | Me | $CH_2CH_2CH_2Si(Me)_3$ | H | $CF_3$ | m.p. 165-169°C |
| C-4 | Me | $CH_2$(furan-2-yl) | H | $CF_3$ | m.p. 117-120°C |
| C-5 | Me | $CH_2$(4-ClPh) | H | $CF_3$ | m.p. 144-149°C |
| C-6 | Me | $CH_2CH_2CH_2COMe$ | H | $CF_3$ | * |
| C-7 | Me | $CH_2CH_2CN$ | H | $CF_3$ | m.p. 171-175°C |
| C-8 | Me | $CH_2CH_2NHCOMe$ | H | $CF_3$ | m.p. 146-150°C |
| C-9 | Me | $CH_2C\equiv CH$ | H | $CF_3$ | m.p. 183-185°C |
| C-10 | Me | $CH_2CH_2SO_2Me$ | H | $CF_3$ | m.p. 119-123°C |
| C-11 | Me | $CH_2CH(OMe)_2$ | H | $CF_3$ | * |
| C-12 | Me | $CH_2CO_2Me$ | H | $CF_3$ | * |
| C-13 | Me | $CH_2CH_2OMe$ | H | $CF_3$ | m.p. 148-150°C |
| C-14 | Me | $CH_2{}^cPr$ | H | $CF_3$ | m.p. 69-70°C |
| C-15 | Me | $^iPr$ | H | $CF_3$ | m.p. 207-208°C |
| C-16 | Me | Et | H | $CF_3$ | m.p. 160-161°C |
| C-17 | Me | Me | H | CN | m.p. 196-198°C |
| C-18 | $MeOCH_2CH_2$ | Me | H | $CF_3$ | m.p. 100-101°C |
| C-19 | Me | Me | H | SOMe | m.p. 182-183°C |
| C-20 | Me | Me | H | $CHF_2$ | m.p. 188-189°C |
| C-21 | $MeSCH_2$ | Me | H | $CF_3$ | m.p. 188-189°C |
| C-22 | Me | Me | H | $SO_2Me$ | m.p. 157-159°C |
| C-23 | $^tBu$ | Me | H | $CF_3$ | * |
| C-24 | Me | Me | H | OMe | 184-186°C |
| C-25 | Me | Me | H | F | m.p. 177-178°C |
| C-26 | Me | Me | H | Me | * |
| C-27 | Me | Me | H | Cl | m.p. 195-197°C |
| C-28 | $CF_3CH_2$ | Me | H | $CF_3$ | m.p. 177-178°C |
| C-29 | Me | Me | $^cPr$ | $CF_3$ | m.p. 176-177°C |
| C-30 | Me | Me | Me | $CF_3$ | * |
| C-31 | $MeOCH_2$ | Me | H | $CF_3$ | * |
| C-32 | Me | $CH_2CH=C(Me)_2$ | Me | $CF_3$ | m.p. 143-147°C |
| C-33 | Me | Me | Me | $OCF_3$ | m.p. 190-191°C |
| C-34 | Me | Me | Me | $OCH_2F$ | m.p. 180-181°C |
| C-35 | Me | $CH_2CF_2CF_3$ | Me | $CF_3$ | * |
| C-36 | Me | $CH_2COPh$ | Me | $CF_3$ | * |

Among the compounds described in Tables 1 to 3, the compounds marked with asterisk (*) in physical property value column were compounds having properties of amorphous or viscous oil. The $^1$H-NMR data thereof are shown below.

Compound A-10: $^1$H-NMR (400MHz, $CDCl_3$): δ3.08 (s, 3H), 3.50 (s, 3H), 3.76 (s, 3H), 4.03 (s, 3H), 7.97 (d, 1H), 7.65-7.68 (m, 2H), 7.74 (t, 1H), 7.82 (d, 1H), 8.01 (d, 1H).

Compound A-11: $^1$H-NMR (400MHz, $CDCl_3$): δ1.59 (s, 9H), 3.54 (s, 3H), 3.72 (s, 3H), 3.91 (s, 3H), 7.31 (s, 1H), 7.73 (m, 2H).

Compound A-15: $^1$H-NMR (400MHz, $CDCl_3$): δ2.78 (s, 3H), 3.14 (s, 3H), 3.25 (s, 3H), 3.52 (s, 3H), 3.70 (s, 3H), 7.21 (s, 1H), 7.76 (d, 1H), 8.07 (d, 1H).

Compound A-17: $^1$H-NMR (400MHz, $CDCl_3$): δ3.28 (s, 3H), 3.42 (s, 3H), 3.49 (s, 3H), 3.66 (s, 3H), 3.77-3.81 (m, 2H), 4.36-4.40 (m, 2H), 7.20 (s, 1H), 7.48 (d, 1H), 7.93 (d, 1H).

Compound A-20: $^1$H-NMR (400MHz, $CDCl_3$): δ2.44-2.56 (m, 2H), 3.10 (s, 3H), 3.75 (s, 3H), 3.79 (t, 2H), 4.64 (s, 2H), 7.19 (s, 1H), 7.72 (dd, 1H), 7.82 (d, 1H), 7.97 (d, 1H).

Compound A-24: $^1$H-NMR (400MHz, $CDCl_3$): δ2.97 (s, 3H), 3.49 (s, 3H), 3.72 (s, 3H), 6.54 (s, 1H), 7.24 (s, 1H), 7.72 (s, 1H), 7.80 (s, 1H), 7.87 (d, 1H), 8.21 (d, 1H).

Compound A-25: $^1$H-NMR (400MHz, $CDCl_3$): δ2.52-2.63 (m, 4H), 3.45-3.67 (m, 15H), 7.19 (s, 1H), 7.70 (d, 1H), 8.16 (d, 1H).

Compound A-26: $^1$H-NMR (400MHz, $CDCl_3$): δ3.14 (s, 3H), 3.53 (s, 3H), 3.79 (s, 3H), 7.42-7.53 (m, 4H), 7.63-7.65 (m, 2H), 7.89 (d, 1H), 7.95 (dd, 1H), 8.22 (d, 1H).

Compound A-28: $^1$H-NMR (400MHz, $CDCl_3$): δ3.10 (s, 3H), 3.49 (s, 3H), 3.51 (s, 3H), 3.74 (s, 3H), 4.56 (s, 2H), 7.20 (s, 1H), 7.71 (dd, 1H), 7.81 (d, 1H), 7.98 (d, 1H).

Compound A-31: $^1$H-NMR (400MHz, $CDCl_3$): δ3.50 (s, 3H), 3.68-3.77 (m, 10H), 4.01-4.14 (m, 4H), 7.20 (s, 1H), 7.61- 7.63 (m, 2H), 8.97 (s, 1H).

Compound A-32: $^1$H-NMR (400MHz, $CDCl_3$): δ2.10 (s, 3H), 3.42-3.50 (m, 7H), 3.66-3.74 (m, 7H), 6.28 (s, 1H), 7.23 (s, 3H), 7.52-7.58 (m, 2H).

Compound A-33: $^1$H-NMR (400MHz, $CDCl_3$): δ2.21-2.46 (m, 2H), 2.52-2.58 (m, 2H), 3.14 (s, 3H), 3.50 (s, 3H), 3.67-3.88 (m, 5H), 7.25 (s, 1H), 7.75 (d, 1H), 8.06-8.11 (m, 1H).

Compound A-35: $^1$H-NMR (400MHz, $CDCl_3$): δ2.28 (s, 3H), 3.17 (s, 3H), 3.53 (s, 3H), 3.71 (s, 3H), 3.96-4.05 (m, 2H), 5.10-5.31 (m, 2H), 7.17 (s, 1H), 7.73 (d, 1H), 8.08 (d, 1H).

Compound A-38: $^1$H-NMR (400MHz, $CDCl_3$): δ2.72 (s, 3H), 3.23 (s, 3H), 3.50 (s, 3H), 3.67 (s, 3H), 4.74-4.75 (m, 2H), 6.93 (s, 1H), 7.20 (s, 1H), 7.55 (d, 1H), 7.97 (d, 1H).

Compound A-40: $^1$H-NMR (400MHz, $CDCl_3$): δ3.52 (s, 3H), 3.70 (s, 3H), 3.73-3.81 (m, 6H), 4.25 (dd, 2H), 7.27 (s, 1H), 7.51 (s, 2H), 9.05 (s, 1H).

Compound A-41: $^1$H-NMR (400MHz, $CDCl_3$): δ2.05 (br, 3H), 2.11 (s, 3H), 2.28 (s, 3H), 3.51 (s, 3H), 3.68 (s, 3H), 3.96 (s, 3H), 7.13 (br, 1H), 7.19 (s, 1H), 7.39 (br, 1H).

Compound A-42: $^1$H-NMR (400MHz, $CDCl_3$): δ3.34 (s, 3H), 3.49 (s, 3H), 3.74 (s, 3H), 7.21 (s, 1H), 7.90 (d, 1H), 8.05 (d, 1H).

Compound A-43: $^1$H-NMR (400MHz, $CDCl_3$): δ2.55-2.68 (m, 6H), 3.15 (s, 3H), 3.65-3.67 (m, 6H), 7.28 (s, 1H), 7.60 (d, 1H), 7.96 (d, 1H).

Compound A-45: $^1$H-NMR (400MHz, $CDCl_3$): δ1.78 (s, 3H), 1.80 (s, 3H), 3.33 (s, 3H), 3.51 (s, 3H), 3.66 (s, 3H), 5.62 (d, 1H), 5.72 (d, 1H), 7.25 (s, 1H), 7.73 (d, 1H), 8.15 (d, 1H).

Compound A-47: $^1$H-NMR (400MHz, $CDCl_3$): δ3.11 (s, 3H), 3.74 (s, 3H), 4.15-4.26 (m, 1H), 4.70-4.80 (m, 1H), 7.25 (s, 1H), 7.96-8.03 (m, 2H), 8.25 (s, 1H).

Compound A-49: $^1$H-NMR (400MHz, $CDCl_3$): δ1.82 (s, 3H), 2.69 (s, 3H), 3.51 (s, 3H), 3.74 (s, 3H), 7.18-7.29 (m, 3H), 7.45-7.49 (m, 2H), 7.75 (d, 1H), 8.17 (d, 1H).

Compound A-50: $^1$H-NMR (400MHz, $CDCl_3$): δ0.76 (t, 3H), 0.87-0.95 (m, 2H), 1.02-1.15 (m, 4H), 1.36-1.48 (m, 2H), 3.17 (s, 3H), 3.52 (s, 3H), 3.75-3.86 (m, 2H), 4.46-4.56 (m, 2H), 7.21 (s, 1H), 7.98-8.02 (m, 2H), 8.29 (s, 1H).

Compound A-52: $^1$H-NMR (400MHz, $CDCl_3$): δ3.54 (s, 3H), 3.71 (s, 3H), 7.31 (s, 1H), 7.68-7.75 (m, 2H), 7.85-7.89 (m, 1H), 8.14 (s, 1H), 8.61 (s, 1H).

Compound A-53: $^1$H-NMR (400 MHz, $CDCl_3$): δ3.30 (s, 3H), 3.43 (s, 3H), 3.80 (s, 3H), 7.36 (dd, 1H), 7.49 (s, 1H), 7.54-7.60 (m, 1H), 7.91 (dd, 1H).

Compound A-54: $^1$H-NMR (400 MHz, $CDCl_3$): δ0.70 (t, 3H), 1.45-1.54 (m, 2H), 3.10 (s, 3H), 3.38 (s, 3H), 3.77 (s, 3H), 3.93 (t, 2H), 7.90-7.97 (m, 2H), 8.21 (s, 1H).

Compound A-56: $^1$H-NMR (400MHz, $CDCl_3$): δ3.54 (s, 3H), 3.69 (s, 3H), 7.30 (s, 1H), 7.60-7.70 (m, 3H), 7.81 (d, 1H), 7.94 (s, 1H).

Compound A-60: $^1$H-NMR (400MHz, $CDCl_3$): δ1.08 (t, 3H), 3.17 (s, 3H), 3.52 (s, 3H), 3.86-3.94 (m, 1H), 4.46-4.54 (m, 1H), 5.21 (d, 1H), 5.25 (d, 1H), 7.20 (s, 1H), 7.94-8.03 (m, 2H), 8.29 (s, 1H).

Compound A-61: $^1$H-NMR (400MHz, $CDCl_3$): δ0.32-0.37 (m, 2H), 0.50-0.56 (m, 2H), 1.19-1.25 (m, 1H), 3.14 (s, 3H), 3.66-3.87 (m, 5H), 7.20 (s, 1H), 7.97-8.01 (m, 2H), 8.27 (s, 1H).

Compound A-62: $^1$H-NMR (400MHz, $CDCl_3$): δ3.13 (s, 3H), 3.58 (s, 3H), 4.23-4.32 (m, 1H), 4.81-4.90 (m, 1H), 7.24 (s, 1H), 7.95-8.05 (m, 2H), 8.28 (s, 1H).
Compound A-63: $^1$H-NMR (400MHz, $CDCl_3$): δ1.08 (t, 3H), 3.18 (s, 3H), 3.43 (s, 3H), 3.87-3.95 (m, 1H), 4.46-4.54 (m, 1H), 5.21 (d, 1H), 5.25 (d, 1H), 7.26 (s, 1H), 7.98-8.04 (m, 2H), 8.29 (s, 1H).
Compound A-64: $^1$H-NMR (400MHz, $CDCl_3$): δ3.16 (s, 3H), 3.43 (s, 3H), 3.80 (s, 3H), 5.20 (d, 1H), 5.27 (d, 1H), 7.27 (s, 1H), 7.99-8.07 (m, 2H), 8.29 (s, 1H).
Compound A-69: $^1$H-NMR (400MHz, $CDCl_3$): δ2.86-3.02 (m, 3H), 3.10-3.44 (m, 4H), 3.53 (s, 3H), 3.76 (s, 3H), 7.21 (s, 1H), 7.55-7.74 (m, 3H).
Compound A-70: $^1$H-NMR (400MHz, $CDCl_3$): δ3.02 (s, 3H), 3.54 (s, 3H), 3.76 (s, 3H), 6.86-6.96 (m, 1H), 7.15 (s, 1H), 7.50-7.82 (m, 4H).
Compound A-71: $^1$H-NMR (400MHz, $CDCl_3$): δ1.45 (t, 3H), 3.20 (q, 2H), 3.35 (s, 3H), 3.48 (s, 3H), 3.65 (s, 3H) , 4.98-5.85 (m, 1H), 7.25 (s, 1H), 7.79 (d, 1H), 8.12 (d, 1H).
Compound A-74: $^1$H-NMR (400MHz, $CDCl_3$): δ3.19-3.30 (m, 4H), 3.52 (s, 3H), 3.69-3.79 (m, 7H), 7.25 (s, 1H), 7.92- 8.09 (m, 3H).
Compound A-75: $^1$H-NMR (400MHz, $CDCl_3$): δ3.13 (s, 3H), 3.51 (s, 3H), 3.78 (s, 3H), 7.20 (s, 1H), 7.59 (dd, 1H) , 7.86 (d, 1H), 7.89 (d, 1H).
Compound B-2: $^1$H-NMR (400MHz, $CDCl_3$): δ2.49-2.63 (m, 2H), 2.80-2.89 (m, 1H), 3.09-3.18 (m, 1H), 3.35-3.40 (m, 1H), 3.55 (s, 3H), 3.69-3.73 (m, 1H), 4.07-4.16 (m, 2H), 4.29-4.34 (m, 1H), 4.61-4.68 (m, 1H), 7.19 (s, 1H), 7.60-7.99 (m, 6H).
Compound B-9: $^1$H-NMR (400MHz, $CDCl_3$): δ1.79 (m, 2H), 2.09-2.23 (m, 2H), 3.30-3.45 (m, 4H), 3.54 (s, 3H), 3.74 (s, 3H), 7.16 (s, 1H), 7.89 (s, 2H).
Compound B-11: $^1$H-NMR (400MHz, $CDCl_3$): δ2.15 (s, 3H), 2.50-2.78 (m, 5H), 3.41-3.56 (m, 5H), 3.69 (s, 3H), 4.12- 4.28 (m, 4H), 7.13 (s, 1H), 7.49 (s, 1H).
Compound B-12: $^1$H-NMR (400MHz, $CDCl_3$): δ1.41 (s, 6H), 2.22-2.40 (m, 4H), 2.56 (s, 3H), 3.39-3.45 (m, 2H), 3.51 (s, 3H), 3.68 (s, 3H), 7.15 (s, 1H), 7.36 (d, 1H), 7.62 (d, 1H).
Compound B-13: $^1$H-NMR (400MHz, $CDCl_3$): δ3.51 (s, 3H), 3.59 (dt, 2H), 3.73 (s, 3H), 4.83-4.95 (m, 2H), 7.18 (s, 1H), 7.56 (d, 1H), 7.76 (d, 1H).
Compound B-15: $^1$H-NMR (400MHz, $CDCl_3$): δ2.46-2.49 (m, 3H), 3.45-3.46 (m, 3H), 3.78-3.79 (m, 3H), 4.10-4.13 (m, 3H), 6.76-6.80 (m, 2H), 7.00-7.05 (m, 3H), 7.38-7.44 (m, 1H), 7.85-7.95 (m, 1H).
Compound C-6: $^1$H-NMR (400MHz, $CDCl_3$): δ1.67-1.86 (m, 4H), 1.97 (s, 3H), 2.52-2.61 (m, 2H), 2.80-2.84 (m, 2H), 3.40-3.47 (m, 2H), 3.53 (s, 3H), 3.79-3.82 (m, 1H), 4.35-4.39 (m, 1H), 7.15 (s, 1H), 7.85-7.88 (m, 2H).
Compound C-11: $^1$H-NMR (400MHz, $CDCl_3$): δ2.49-2.52 (m, 2H), 2.82-2.88 (m, 2H), 3.12 (s, 3H), 3.21 (s, 3H), 3.36- 3.46 (m, 2H), 3.54 (s, 3H), 3.78-3.82 (m, 1H), 4.15-4.30 (m, 2H), 7.16 (s, 1H), 7.89 (s, 2H).
Compound C-12: $^1$H-NMR (400MHz, $CDCl_3$): δ2.50-2.55 (m, 2H), 2.83-2.96 (m, 2H), 3.36-3.43 (m, 2H), 3.54 (s, 3H), 3.66 (s, 3H), 4.74 (s, 2H), 7.17 (s, 1H), 7.86-7.91 (m, 2H).
Compound C-23: $^1$H-NMR (400MHz, $CDCl_3$): δ1.52 (s, 9H), 2.46-2.50 (m, 2H), 2.74-2.88 (m, 2H), 3.34-3.37 (m, 2H), 3.67 (s, 3H), 7.05 (s, 1H), 7.83-7.86 (m, 2H).
Compound C-26: $^1$H-NMR (400MHz, $CDCl_3$): δ2.37-2.42 (m, 2H), 2.62-2.78 (m, 2H), 2.80 (s, 3H), 3.37-3.40 (m, 2H), 3.52 (s, 3H), 3.70 (s, 3H), 7.14 (s, 1H), 7.28 (d, 1H), 7.58 (d, 1H).
Compound C-30: $^1$H-NMR (400MHz, $CDCl_3$): δ1.87 (s, 3H), 2.44-2.65 (m, 2H), 3.06-3.15 (m, 2H), 3.38-3.53 (m, 5H), 3.72 (s, 3H), 7.38 (d, 1H), 7.80 (d, 1H).
Compound C-31: $^1$H-NMR (400MHz, $CDCl_3$): δ2.48-2.98 (m, 4H), 3.36-3.45 (m, 5H), 3.74 (s, 3H), 5.10 (d, 1H), 5.34 (d, 1H), 7.21 (d, 1H), 7.83-7.90 (m, 2H).
Compound C-35: $^1$H-NMR (400MHz, $CDCl_3$): δ2.45-2.52 (m, 2H), 2.67-2.83 (m, 2H), 3.29-3.42 (m, 2H), 3.54 (s, 3H), 4.31 (dt, 1H), 4.86-4.95 (m, 1H), 7.21 (s, 1H), 7.79-7.96 (m, 2H).
Compound C-36: $^1$H-NMR (400MHz, $CDCl_3$): δ2.49-2.51 (m, 2H), 2.79-2.83 (m, 1H), 2.93-2.99 (m, 1H), 3.32-3.37 (m, 2H), 3.54 (s, 3H), 5.01-5.17 (m, 1H), 5.47-5.53 (m, 1H), 7.16 (s, 1H), 7.30-7.36 (m, 2H), 7.51-7.77 (m, 5H).

(Evaluation of herbicidal effects)

Next, the following test examples show that the compound of the present invention is useful as an active ingredient of a herbicide.

(Test Example 1)

(1) Preparation of test emulsion

POA allylphenyl ether (4.1 parts by weight), POE-POP glycol (1 part by weight), POE sorbitan laurate (0.8 parts

by weight), glycerin (2.6 parts by weight), dimethylformamide (65.9 parts by weight), N-methylpyrrolidone (5.1 parts by weight), cyclohexanone (15.4 parts by weight), and aromatic hydrocarbons (5.1 parts by weight) were mixed and dissolved to prepare an emulsion. The compound of the present invention (4 mg) was dissolved in this emulsion (100 μL) to prepare a test emulsion. POA means "polyoxyalkylene", POE means "polyoxyethylene", and POP means "polyoxypropylene".

(2) Foliar spraying treatment

A 150 $cm^2$ pot was filled with soil, and seeds of Avena sativa, Matricaria chamomilla, Setaria faberi, Digitaria ciliaris, Abutilon theophrasti and Amaranthus retroflexus were sown into the surface layer which was covered lightly with soil. Thereafter, they were grown in a greenhouse. When each plant grew to a plant height of 2 to 4 cm, the above test emulsion was diluted so that the amount of the active ingredient became a predetermined amount, and sprayed on the foliage with a small sprayer at a spray water volume of 250 L per hectare.

(3) Evaluation

After 3 weeks, above ground weights of weeds in the untreated and treated areas were measured for each weed, and the weed killing rate was calculated by the following calculation formula.

(4) Calculation formula for weed killing rate

Weed killing rate (%) = [(above ground weight of weeds in untreated area) - (above ground weight of weeds in treated area) / (above ground weight of weeds in untreated area)] × 100

The compounds of A-1, A-2, A-3, A-4, A-5, A-6, A-9, A-10 and B-1 were applied so that the spray volume was 1,000 g per hectare. As a result, all the compounds had a weed killing rate of 80% or more with respect to Abutilon theophrasti.

The compounds of A-1, A-2, A-3, A-4, A-5, A-6, A-10 and B-1 were applied so that the spray volume was 1,000 g per hectare. As a result, all the compounds had a weed killing rate of 80% or more with respect to Matricaria chamomilla.

The evaluation results will be further shown in succession.

(a) Abutilon theophrasti

The compounds shown in Table 4 were applied so that the spray volume was 1,000 g per hectare. As a result, all the compounds showed a herbicidal activity of 80% or more with respect to Abutilon theophrasti.

Table 4

| Compound number | | | | |
|---|---|---|---|---|
| A-1 | A-39 | A-58 | B-8 | C-13 |
| A-2 | A-42 | A-59 | B-9 | C-14 |
| A-4 | A-43 | A-60 | B-10 | C-16 |
| A-5 | A-46 | A-61 | B-11 | C-26 |
| A-9 | A-47 | A-62 | B-12 | C-27 |
| A-10 | A-48 | A-63 | B-13 | C-29 |
| A-33 | A-49 | A-64 | B-16 | C-30 |
| A-34 | A-50 | B-1 | C-10 | C-31 |

(continued)

| Compound number | | | | |
|---|---|---|---|---|
| A-35 | A-54 | B-6 | C-11 | |
| A-37 | A-57 | B-7 | C-12 | |

The compounds shown in Table 5 were applied so that the spray volume was 250 g per hectare. As a result, all the compounds showed a herbicidal activity of 80% or more with respect to Abutilon theophrasti.

Table 5

| Compound number | | | | | | | |
|---|---|---|---|---|---|---|---|
| A-1 | A-18 | A-39 | A-60 | B-2 | C-2 | C-13 | C-30 |
| A-2 | A-20 | A-42 | A-61 | B-3 | C-3 | C-14 | C-31 |
| A-3 | A-21 | A-46 | A-62 | B-5 | C-4 | C-15 | C-32 |
| A-4 | A-22 | A-47 | A-63 | B-6 | C-5 | C-16 | C-33 |
| A-5 | A-24 | A-48 | A-64 | B-9 | C-6 | C-18 | C-34 |
| A-10 | A-26 | A-49 | A-71 | B-10 | C-7 | C-20 | C-35 |
| A-13 | A-28 | A-50 | A-74 | B-11 | C-9 | C-23 | C-36 |
| A-15 | A-30 | A-57 | A-75 | B-13 | C-10 | C-24 | |
| A-16 | A-33 | A-58 | A-77 | B-15 | C-11 | C-26 | |
| A-17 | A-35 | A-59 | B-1 | C-1 | C-12 | C-29 | |

(b) Matricaria chamomilla

The compounds shown in Table 6 were applied so that the spray volume was 1,000 g per hectare. As a result, all the compounds showed a herbicidal activity of 80% or more with respect to Matricaria chamomilla.

Table 6

| Compound number | | | | |
|---|---|---|---|---|
| A-1 | A-43 | A-62 | B-13 | C-28 |
| A-2 | A-46 | A-63 | B-16 | C-29 |
| A-4 | A-47 | A-64 | C-10 | C-30 |
| A-5 | A-48 | B-1 | C-11 | C-31 |
| A-10 | A-50 | B-6 | C-12 | |
| A-33 | A-57 | B-7 | C-13 | |
| A-34 | A-58 | B-9 | C-14 | |
| A-35 | A-59 | B-10 | C-16 | |
| A-39 | A-60 | B-11 | C-26 | |
| A-42 | A-61 | B-12 | C-27 | |

The compounds shown in Table 7 were applied so that the spray volume was 250 g per hectare. As a result, all the compounds showed a herbicidal activity of 80% or more with respect to Matricaria chamomilla.

Table 7

| Compound number | | | | | | | | |
|---|---|---|---|---|---|---|---|---|
| A-1 | A-18 | A-33 | A-50 | A-75 | B-15 | C-11 | C-25 | C-36 |
| A-2 | A-19 | A-34 | A-57 | A-77 | C-1 | C-12 | C-26 | |
| A-3 | A-20 | A-35 | A-58 | B-1 | C-2 | C-13 | C-27 | |
| A-4 | A-21 | A-39 | A-59 | B-2 | C-3 | C-14 | C-28 | |
| A-5 | A-22 | A-42 | A-60 | B-5 | C-4 | C-16 | C-30 | |
| A-10 | A-26 | A-43 | A-61 | B-6 | C-5 | C-18 | C-31 | |
| A-13 | A-28 | A-45 | A-62 | B-7 | C-6 | C-19 | C-32 | |
| A-15 | A-29 | A-46 | A-63 | B-9 | C-7 | C-20 | C-33 | |
| A-16 | A-30 | A-47 | A-64 | B-10 | C-9 | C-23 | C-34 | |
| A-17 | A-32 | A-48 | A-71 | B-13 | C-10 | C-24 | C-35 | |

(c) Avena sativa

The compounds shown in Table 8 were applied so that the spray volume was 1,000 g per hectare. As a result, all the compounds showed a herbicidal activity of 80% or more with respect to Avena sativa.

Table 8

| Compound number | |
|---|---|
| A-1 | B-9 |
| A-2 | B-16 |
| A-10 | C-10 |
| A-33 | C-11 |
| A-42 | C-13 |
| A-46 | C-14 |
| A-49 | C-26 |
| A-50 | C-30 |
| A-58 | C-31 |
| A-60 | |

The compounds shown in Table 9 were applied so that the spray volume was 250 g per hectare. As a result, all the compounds showed a herbicidal activity of 80% or more with respect to Avena sativa.

Table 9

| Compound number | | |
|---|---|---|
| A-1 | C-3 | C-30 |
| A-10 | C-4 | C-32 |
| A-16 | C-5 | C-34 |
| A-21 | C-6 | C-35 |
| A-31 | C-7 | |
| A-33 | C-10 | |
| A-50 | C-13 | |

(continued)

| Compound number | | |
|---|---|---|
| A-58 | C-18 | |
| B-5 | C-20 | |
| B-15 | C-26 | |

(d) Setaria faberi

The compounds shown in Table 10 were applied so that the spray volume was 1,000 g per hectare. As a result, all the compounds showed a herbicidal activity of 80% or more with respect to Setaria faberi.

Table 10

| Compound number | | | |
|---|---|---|---|
| A-1 | A-48 | B-1 | C-14 |
| A-2 | A-49 | B-7 | C-16 |
| A-4 | A-50 | B-9 | C-26 |
| A-10 | A-58 | B-11 | C-27 |
| A-33 | A-59 | B-14 | C-29 |
| A-35 | A-60 | B-16 | C-30 |
| A-42 | A-61 | C-10 | C-31 |
| A-43 | A-62 | C-11 | |
| A-46 | A-63 | C-12 | |
| A-47 | A-64 | C-13 | |

The compounds shown in Table 11 were applied so that the spray volume was 250 g per hectare. As a result, all the compounds showed a herbicidal activity of 80% or more with respect to Setaria faberi.

Table 11

| Compound number | | | | | | |
|---|---|---|---|---|---|---|
| A-1 | A-33 | A-60 | B-5 | C-8 | C-19 | C-36 |
| A-2 | A-35 | A-62 | B-9 | C-9 | C-20 | |
| A-3 | A-42 | A-63 | B-15 | C-10 | C-26 | |
| A-10 | A-43 | A-64 | C-1 | C-11 | C-27 | |
| A-16 | A-46 | A-71 | C-2 | C-12 | C-29 | |
| A-21 | A-47 | A-75 | C-3 | C-13 | C-30 | |
| A-22 | A-48 | B-1 | C-4 | C-14 | C-31 | |
| A-23 | A-50 | B-2 | C-5 | C-15 | C-32 | |
| A-30 | A-58 | B-3 | C-6 | C-16 | C-34 | |
| A-31 | A-59 | B-4 | C-7 | C-18 | C-35 | |

(e) Digitaria ciliaris

The compounds shown in Table 12 were applied so that the spray volume was 1,000 g per hectare. As a result, all the compounds showed a herbicidal activity of 80% or more with respect to Digitaria ciliaris.

Table 12

| Compound number | | | |
|---|---|---|---|
| A-1 | A-47 | A-64 | C-10 |
| A-2 | A-49 | B-1 | C-11 |
| A-4 | A-50 | B-6 | C-12 |
| A-10 | A-57 | B-7 | C-13 |
| A-33 | A-58 | B-9 | C-14 |
| A-35 | A-59 | B-11 | C-16 |
| A-39 | A-60 | B-12 | C-26 |
| A-42 | A-61 | B-13 | C-27 |
| A-43 | A-62 | B-14 | C-30 |
| A-46 | A-63 | B-16 | C-31 |

The compounds shown in Table 13 were applied so that the spray volume was 250 g per hectare. As a result, all the compounds showed a herbicidal activity of 80% or more with respect to Digitaria ciliaris.

Table 13

| Compound number | | | | | | |
|---|---|---|---|---|---|---|
| A-1 | A-21 | A-48 | A-64 | C-2 | C-13 | C-30 |
| A-2 | A-22 | A-49 | A-71 | C-3 | C-14 | C-31 |
| A-3 | A-24 | A-50 | A-75 | C-4 | C-16 | C-32 |
| A-10 | A-25 | A-57 | B-1 | C-5 | C-18 | C-34 |
| A-13 | A-31 | A-58 | B-2 | C-6 | C-19 | C-35 |
| A-15 | A-33 | A-59 | B-5 | C-7 | C-20 | C-36 |
| A-16 | A-35 | A-60 | B-6 | C-8 | C-24 | |
| A-17 | A-42 | A-61 | B-9 | C-10 | C-25 | |
| A-18 | A-45 | A-62 | B-11 | C-11 | C-26 | |
| A-19 | A-47 | A-63 | B-15 | C-12 | C-27 | |

(f) Amaranthus retroflexus

The compounds shown in Table 14 were applied so that the spray volume was 1,000 g per hectare. As a result, all the compounds showed a herbicidal activity of 80% or more with respect to Amaranthus retroflexus.

Table 14

| Compound number | | | | | |
|---|---|---|---|---|---|
| A-1 | A-36 | A-51 | A-65 | B-14 | C-28 |
| A-2 | A-37 | A-56 | B-1 | B-16 | C-29 |
| A-4 | A-39 | A-57 | B-6 | C-10 | C-30 |
| A-5 | A-42 | A-58 | B-7 | C-11 | C-31 |
| A-6 | A-43 | A-59 | B-8 | C-12 | |
| A-9 | A-44 | A-60 | B-9 | C-13 | |

(continued)

| Compound number | | | | | |
|---|---|---|---|---|---|
| A-10 | A-46 | A-61 | B-10 | C-14 | |
| A-33 | A-47 | A-62 | B-11 | C-16 | |
| A-34 | A-49 | A-63 | B-12 | C-26 | |
| A-3 5 | A-50 | A-64 | B-13 | C-27 | |

The compounds shown in Table 15 were applied so that the spray volume was 250 g per hectare. As a result, all the compounds showed a herbicidal activity of 80% or more with respect to Amaranthus retroflexus.

Table 15

| Compound number | | | | | | | | |
|---|---|---|---|---|---|---|---|---|
| A-1 | A-19 | A-3 5 | A-54 | A-75 | B-11 | C-7 | C-17 | C-33 |
| A-2 | A-20 | A-37 | A-57 | A-77 | B-13 | C-8 | C-18 | C-35 |
| A-3 | A-21 | A-39 | A-58 | B-1 | B-14 | C-9 | C-20 | C-36 |
| A-4 | A-22 | A-42 | A-59 | B-2 | B-15 | C-10 | C-23 | |
| A-5 | A-24 | A-44 | A-60 | B-3 | C-1 | C-11 | C-26 | |
| A-10 | A-26 | A-45 | A-61 | B-5 | C-2 | C-12 | C-28 | |
| A-15 | A-28 | A-46 | A-62 | B-6 | C-3 | C-13 | C-29 | |
| A-16 | A-30 | A-47 | A-63 | B-8 | C-4 | C-14 | C-30 | |
| A-17 | A-33 | A-49 | A-64 | B-9 | C-5 | C-15 | C-31 | |
| A-18 | A-34 | A-50 | A-71 | B-10 | C-6 | C-16 | C-32 | |

Since all of those randomly selected from among the compounds of the present invention exert the above-mentioned effects, it can be understood that the compounds of the present invention including the compounds that are not exemplified are compounds having high herbicidal effects.

While preferred embodiments of the invention have been described and illustrated above, it should be understood that these are exemplary of the invention and are not to be considered as limiting. Accordingly, the invention is not to be considered as being limited by the foregoing description, and is only limited by the scope of the appended claims.

INDUSTRIAL APPLICABILITY

It is possible to provide a novel 7-oxa-3,4-diazabicyclo [4.1.0] hept-4-en-2-one compound useful as an active ingredient of a herbicide, which has a reliable weed control effect even at a low dose, has less phytotoxicity to crops, and is highly safe for the environment; and a herbicide.

## Claims

**1.** A compound represented by a formula (I) or a salt thereof:

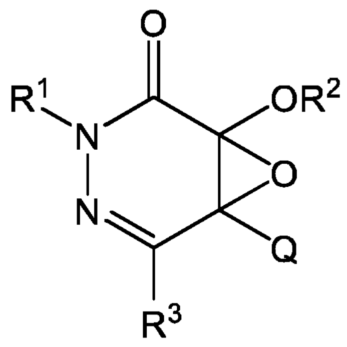

(I)

wherein

$R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group, or a 5- to 6-membered cyclic ether group,
$R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group,
$R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group, or a substituted or unsubstituted phenyl group, and
Q represents a substituted or unsubstituted phenyl group or a substituted or unsubstituted naphthyl group.

**2.** The compound represented by the formula (I) according to Claim 1 which is represented by a formula (I-1), or a salt thereof:

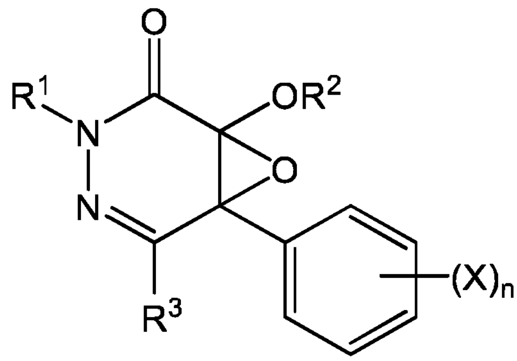

(I-1)

wherein $R^1$, $R^2$, and $R^3$ are the same as defined in Claim 1,
X represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a substituted or unsubstituted $C_{2-6}$ alkenyloxy group, a substituted or unsubstituted $C_{2-6}$ alkynyloxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted $C_{3-6}$ cycloalkyloxy group, a substituted or unsubstituted phenyl group, a phenoxy group, a substituted or unsubstituted 5- to 6-membered heterocyclyl group, a substituted or unsubstituted 5- to 6-membered heterocyclyloxy group, a substituted or unsubstituted phenylsulfonyl group, a group represented by R-CO-, a group represented by RO-CO-, a group represented by R-CONR$^a$-, a group represented by RNH-CO-, a group represented by $R_2N$-CO-, a group represented by RO-CO-NR$^a$-, a group represented by RNH-CO-NH-, a group represented by $R_2N$-CO-NH-, a group represented by RNH-CO-CO-NH-, a group represented by $R_2N$-CO-CO-NH-, a group represented by R-S(O)$_2$-NH-, a group represented by $R_2N$-S(O)$_2$-, a group represented by $R_2S$(O)=N-, a group represented by R-S(O)(=N-R$^b$)-, a group represented by RO-N=C(R$^c$)-, a nitro group, or a cyano group;
each R independently represents a substituted or unsubstituted $C_{1-6}$ alkyl group or a substituted or unsubstituted $C_{3-6}$ cycloalkyl group,

each $R^a$ independently represents a hydrogen atom, a substituted or unsubstituted $C_{1-6}$ alkyl group, or a substituted or unsubstituted $C_{1-6}$ alkoxy group,
$R^b$ represents a substituted or unsubstituted $C_{1-6}$ alkyl group or a substituted or unsubstituted phenyl group,
$R^c$ represents a hydrogen atom or a substituted or unsubstituted $C_{1-6}$ alkyl group;
in the above group represented by $R_2N$-CO-, the group represented by $R_2N$-CO-NH-, the group represented by $R_2N$-CO-CO-NH-, or the group represented by $R_2N$-$S(O)_2$-, R and R may be bonded to form a 4- to 6-membered ring together with a nitrogen atom to which they are bonded;
in the above group represented by $R_2S(O)$=N-, R and R may be bonded to form a 5- to 6-membered ring together with a sulfur atom to which they are bonded;
n represents an integer of 0 to 5, when n is 2 or more, the X groups may be the same or different, and when n is 2 or more, two of the X groups thereof may be combined to form a divalent organic group.

3. The compound represented by the formula (I) according to Claim 1 which is represented by a formula (I-3), or a salt thereof:

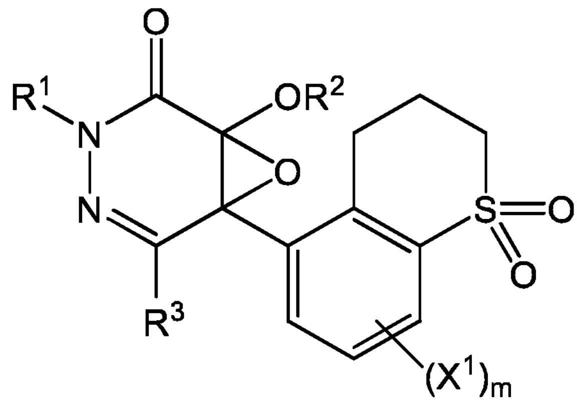

(I-3)

wherein $R^1$, $R^2$, and $R^3$ are the same as defined in Claim 1, wherein $X^1$ represents a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted phenyl group, a substituted or unsubstituted 5- to 6-membered heterocyclyl group, a nitro group, or a cyano group;
m represents an integer of 0 to 3, and when m is 2 or more, the $X^1$ groups may be the same or different.

4. The compound according to Claim 1, or a salt thereof,

wherein a substituent on the $C_{1-6}$ alkyl group represented by $R^2$ is at least one selected from the group consisting of a halogeno group, a hydroxyl group, a $C_{1-6}$ alkoxy group, a $C_{1-6}$ alkoxy $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group, a $C_{3-6}$ cycloalkyl group, a phenyl group, a 5-membered heteroaryl group, a $C_{1-6}$ alkylcarbonyl group, a benzoyl group, a $C_{1-6}$ alkoxycarbonyl group, a $C_{1-6}$ alkylcarboxamide group, a (1,3-dioxoisoindrin-2-yl)oxy group, a trimethylsilyl group and a cyano group, and
a group consisting of a $C_{1-6}$ alkyl group substituted, halogeno group substituted, $C_{1-6}$ haloalkyl group substituted or $C_{1-6}$ haloalkoxy group substituted phenyl group; a $C_{1-6}$ alkyl group substituted, halogeno group substituted, $C_{1-6}$ haloalkyl group substituted or $C_{1-6}$ haloalkoxy group substituted phenoxy group; and a $C_{1-6}$ alkyl group substituted, halogeno group substituted, $C_{1-6}$ haloalkyl group substituted or $C_{1-6}$ haloalkoxy group substituted 5-membered heteroaryl group.

5. A herbicide comprising at least one selected from the group consisting of the compound according to any one of Claims 1 to 4 and a salt thereof as an active ingredient.

6. A method for controlling monocotyledonous and / or dicotyledonous weeds in useful plants, which is a method comprising a step of applying the compound according to any one of Claims 1 to 4 or a salt thereof, or a herbicide

containing said compound to said weed and / or said plant and / or its location.

## Patentansprüche

1. Verbindung, dargestellt durch die Formel (I) oder ein Salz davon:

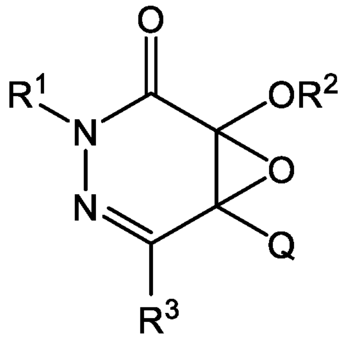

(I)

wobei

$R^1$ eine substituierte oder unsubstituierte $C_{1-6}$ -Alkylgruppe, eine substituierte oder unsubstituierte $C_{2-6}$ -Alkenylgruppe, eine substituierte oder unsubstituierte $C_{2-6}$ - Alkinylgruppe, eine substituierte oder unsubstituierte $C_{3-6}$ -Cycloalkylgruppe oder eine 5-bis 6-gliedrige cyclische Ethergruppe darstellt,
$R^2$ eine substituierte oder unsubstituierte $C_{1-6}$ Alkylgruppe, eine substituierte oder unsubstituierte $C_{2-6}$ Alkenylgruppe oder eine substituierte oder unsubstituierte $C_{2-6}$ Alkynylgruppe darstellt,
$R^3$ ein Wasserstoffatom, eine substituierte oder unsubstituierte $C_{1-6}$ Alkylgruppe, eine substituierte oder unsubstituierte $C_{2-6}$ Alkenylgruppe, eine substituierte oder unsubstituierte $C_{2-6}$ Alkinylgruppe, eine substituierte oder unsubstituierte $C_{1-6}$ Alkoxygruppe, eine substituierte oder unsubstituierte $C_{3-6}$ Cycloalkylgruppe oder eine substituierte oder unsubstituierte Phenylgruppe darstellt, und
Q stellt eine substituierte oder unsubstituierte Phenylgruppe oder eine substituierte oder unsubstituierte Naphthylgruppe dar.

2. Verbindung, dargestellt durch die Formel (I) nach Anspruch 1, die durch die Formel (I-1) dargestellt wird, oder ein Salz davon:

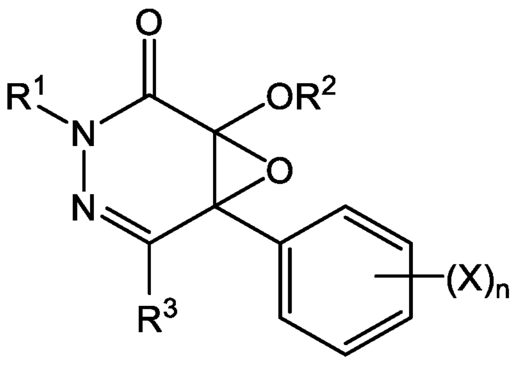

(I-1)

worin $R^1$, $R^2$ und $R^3$ die gleiche Bedeutung wie in Anspruch 1 haben,
X stellt eine Halogengruppe, eine substituierte oder unsubstituierte $C_{1-6}$ Alkylgruppe, eine substituierte oder unsubstituierte $C_{2-6}$ Alkenylgruppe, eine substituierte oder unsubstituierte $C_{2-6}$ Alkinylgruppe, eine Hydroxylgruppe, eine substituierte oder unsubstituierte $C_{1-6}$ Alkoxygruppe, eine substituierte oder unsubstituierte $C_{2-6}$ Alkenyloxygruppe, eine substituierte oder unsubstituierte $C_{2-6}$ Alkinyloxygruppe, eine substituierte oder unsubstituierte $C_{1-6}$ Alkylthiogruppe, eine substituierte oder unsubstituierte $C_{1-6}$ Alkylsulfinylgruppe, eine substituierte oder unsubstituierte $C_{1-6}$ Alkylsulfonylgruppe, eine substituierte oder unsubstituierte $C_{3-6}$ Cycloalkylgruppe, eine substituierte oder unsubstituierte $C_{3-6}$ Cycloalkyloxygruppe, eine substituierte oder unsubstituierte Phenylgruppe, eine Phenoxygruppe, eine substituierte oder unsubstituierte 5- bis 6-gliedrige Heterocyclylgruppe, eine substituierte oder unsubstituierte 5- bis 6-gliedrige Heterocyclyloxygruppe, eine substituierte oder unsubstituierte Phenylsulfonylgruppe, eine Gruppe, dargestellt durch R-CO-, eine Gruppe, dargestellt durch RO-CO-, eine Gruppe, dargestellt durch R-CONR$^a$ -, eine Gruppe, dargestellt durch RNH-CO-, eine Gruppe, dargestellt durch $R_2$ N-CO-, eine Gruppe, dargestellt durch RO-CO-NR$^a$ -, eine Gruppe, dargestellt durch RNH-CO-NH-, eine Gruppe, dargestellt durch $R_2$ N-CO-NH-, eine Gruppe, dargestellt durch RNH-CO-CO-NH-, eine Gruppe, dargestellt durch $R_2$ N-CO-CO-NH-, eine Gruppe, dargestellt durch R-S(O)$_2$ - NH-, eine Gruppe, dargestellt durch $R_2$ N-S(O)$_2$ -, eine Gruppe, dargestellt durch $R_2$ S(O)=N-, eine Gruppe, dargestellt durch R-S(O)(=N-R$^b$ )-, eine Gruppe, dargestellt durch RO-N=C(R$^c$ )-, eine Nitrogruppe oder eine Cyanogruppe;
jedes R unabhängig eine substituierte oder unsubstituierte $C_{1-6}$ Alkylgruppe oder eine substituierte oder unsubstituierte $C_{3-6}$ Cycloalkylgruppe darstellt,
jedes R$^a$ unabhängig ein Wasserstoffatom, eine substituierte oder unsubstituierte $C_{1-6}$ Alkylgruppe oder eine substituierte oder unsubstituierte $C_{1-6}$ Alkoxygruppe darstellt,
R$^b$ steht für eine substituierte oder unsubstituierte $C_{1-6}$ Alkylgruppe oder eine substituierte oder unsubstituierte Phenylgruppe,
R$^c$ ist ein Wasserstoffatom oder eine substituierte oder unsubstituierte $C_{1-6}$ Alkylgruppe;
in der obigen Gruppe, die durch $R_2$ N-CO-, die Gruppe, die durch $R_2$ N-CO-NH-, die Gruppe, die durch $R_2$ N-CO-CO-NH-, oder die Gruppe, die durch $R_2$ N-S(O)$_2$ - dargestellt wird, dargestellt wird, können R und R miteinander verbunden sein, um einen 4- bis 6-gliedrigen Ring zusammen mit einem Stickstoffatom, an das sie gebunden sind, zu bilden;
in der oben durch $R_2$ S(O)=N- dargestellten Gruppe können R und R zusammen mit einem Schwefelatom, an das sie gebunden sind, zu einem 5- bis 6-gliedrigen Ring verbunden sein;
n eine ganze Zahl von 0 bis 5 darstellt, wenn n 2 oder mehr ist, können die X-Gruppen gleich oder verschieden sein, und wenn n 2 oder mehr ist, können zwei der X-Gruppen davon kombiniert werden, um eine zweiwertige organische Gruppe zu bilden.

**3.** Verbindung, dargestellt durch die Formel (I) nach Anspruch 1, die durch die Formel (I-3) dargestellt wird, oder ein Salz davon:

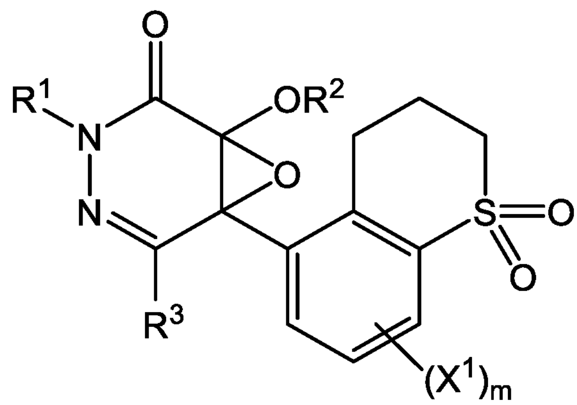

(I-3)

worin R$^1$, R$^2$ und R$^3$ die gleiche Bedeutung wie in Anspruch 1 haben, worin X$^1$ eine Halogengruppe, eine substituierte oder unsubstituierte $C_{1-6}$ Alkylgruppe, eine substituierte oder unsubstituierte $C_{2-6}$ Alkenylgruppe, eine substituierte oder unsubstituierte $C_{2-6}$ Alkinylgruppe, eine substituierte oder unsubstituierte $C_{1-6}$ Alkoxygruppe, eine substituierte oder unsubstituierte $C_{1-6}$ Alkylthiogruppe, eine substituierte oder unsubstituierte $C_{1-6}$ Alkylsulfinylgruppe, eine substituierte oder unsubstituierte $C_{1-6}$ Alkylsulfonylgruppe, eine substituierte oder unsubstituierte $C_{3-6}$ Cycloalkylgruppe, eine substituierte oder unsubstituierte Phenylgruppe, eine substituierte oder unsubstituierte 5- bis 6-gliedrige Heterocyclylgruppe, eine Nitrogruppe oder eine Cyanogruppe;
m ist eine ganze Zahl von 0 bis 3, und wenn m 2 oder mehr ist, können die Gruppen X$^1$ gleich oder verschieden sein.

**4.** Die Verbindung nach Anspruch 1 oder ein Salz davon,

wobei ein Substituent an der durch R$^2$ dargestellten $C_{1-6}$ Alkylgruppe mindestens einer ist, ausgewählt aus der

Gruppe bestehend aus einer Halogengruppe, einer Hydroxylgruppe, einer $C_{1-6}$ Alkoxygruppe, einer $C_{1-6}$ Alkoxy-$C_{1-6}$ Alkoxygruppe, einer $C_{1-6}$ Halogenalkoxygruppe, einer $C_{1-6}$ Alkylthiogruppe, einer $C_{1-6}$ Alkylsulfinylgruppe, eine $C_{1-6}$ Alkylsulfonylgruppe, eine $C_{3-6}$ Cycloalkylgruppe, eine Phenylgruppe, eine 5-gliedrige Heteroarylgruppe, eine $C_{1-6}$ Alkylcarbonylgruppe, eine Benzoylgruppe, eine $C_{1-6}$ Alkoxycarbonylgruppe, eine $C_{1-6}$ Alkylcarboxamidgruppe, eine (1,3-Dioxoisoindrin-2-yl)oxygruppe, eine Trimethylsilylgruppe und eine Cyanogruppe, und eine Gruppe, bestehend aus einer substituierten $C_{1-6}$ Alkylgruppe, einer substituierten Halogengruppe, einer substituierten $C_{1-6}$ Halogenalkylgruppe oder einer substituierten $C_{1-6}$ Halogenalkoxygruppe, einer substituierten $C_{1-6}$ Alkylgruppe, einer substituierten Halogengruppe, einer substituierten $C_{1-6}$ Halogenalkylgruppe oder einer substituierten $C_{1-6}$ Halogenalkoxygruppe, einer Phenoxygruppe und einer substituierten $C_{1-6}$ Alkylgruppe, einer substituierten Halogengruppe, einer substituierten $C_{1-6}$ Halogenalkylgruppe oder einer substituierten $C_{1-6}$ Halogenalkoxygruppe, einer 5-gliedrigen Heteroarylgruppe.

5. Herbizid, umfassend mindestens eine Verbindung, ausgewählt aus der Gruppe, bestehend aus der Verbindung nach einem der Ansprüche 1 bis 4 und einem Salz davon als Wirkstoff.

6. Verfahren zur Bekämpfung von einkeimblättrigen und/oder zweikeimblättrigen Unkräutern in Nutzpflanzen, wobei das Verfahren einen Schritt des Aufbringens der Verbindung nach einem der Ansprüche 1 bis 4 oder eines Salzes davon oder eines Herbizids, das die Verbindung enthält, auf das Unkraut und/oder die Pflanze und/oder ihren Standort umfasst.

**Revendications**

1. Composé représenté par la formule (I) ou sel de celui-ci :

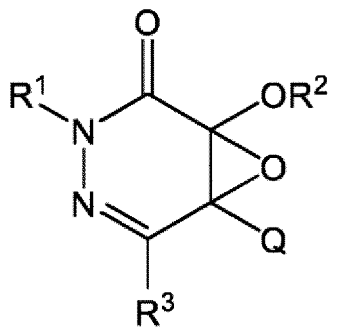

(I)

dans laquelle

$R^1$ représente un groupe alkyle en $C_{1-6}$ substitué ou non substitué, un groupe alcényle en $C_{2-6}$ substitué ou non substitué, un groupe alcynyle en $C_{2-6}$ substitué ou non substitué, un groupe cycloalkyle en $C_{3-6}$ substitué ou non substitué, ou un groupe éther cyclique de 5 à 6 chaînons,
$R^2$ représente un groupe alkyle en $C_{1-6}$ substitué ou non substitué, un groupe alcényle en $C_{2-6}$ substitué ou non substitué, ou un groupe alcynyle en $C_{2-6}$ substitué ou non substitué,
$R^3$ représente un atome d'hydrogène, un groupe alkyle en $C_{1-6}$ substitué ou non substitué, un groupe alcényle en $C_{2-6}$ substitué ou non substitué, un groupe alcynyle en $C_{2-6}$ substitué ou non substitué, un groupe alcoxy en $C_{1-6}$ substitué ou non substitué, un groupe cycloalkyle en $C_{3-6}$ substitué ou non substitué, ou un groupe phényle substitué ou non substitué, et
Q représente un groupe phényle substitué ou non substitué ou un groupe naphtyle substitué ou non substitué.

2. Composé représenté par la formule (I) selon la revendication 1 qui est représenté par une formule (I-1), ou sel de celui-ci :

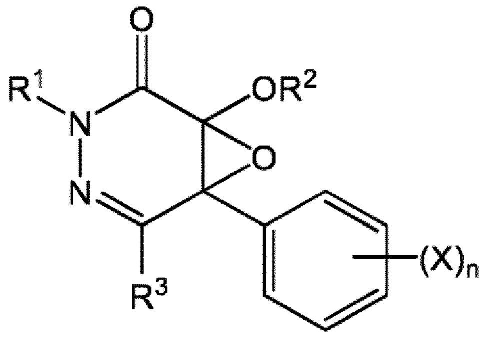

(I-1)

dans laquelle $R^1$, $R^2$ et $R^3$ sont tels que définis dans la revendication 1,

X représente un groupe halogéno, un groupe alkyle en $C_{1-6}$ substitué ou non substitué, un groupe alcényle en $C_{2-6}$ substitué ou non substitué, un groupe alcynyle en $C_{2-6}$ substitué ou non substitué, un groupe hydroxyle, un groupe alcoxy en $C_{1-6}$ substitué ou non substitué, un groupe alcényloxy en $C_{2-6}$ substitué ou non substitué, un groupe alcynyloxy en $C_{2-6}$ substitué ou non substitué, un groupe alkylthio en $C_{1-6}$ substitué ou non substitué, un groupe alkylsulfinyle en $C_{1-6}$ substitué ou non substitué, un groupe alkylsulfonyle en $C_{1-6}$ substitué ou non substitué, un groupe cycloalkyle en $C_{3-6}$ substitué ou non substitué, un groupe cycloalkyloxy en $C_{3-6}$ substitué ou non substitué, un groupe phényle substitué ou non substitué, un groupe phénoxy, un groupe hétérocyclyle de 5 à 6 chaînons substitué ou non substitué, un groupe hétérocyclyloxy de 5 à 6 chaînons substitué ou non substitué, un groupe phénylsulfonyle substitué ou non substitué, un groupe représenté par R-CO-, un groupe représenté par RO-CO-, un groupe représenté par R-CONR$^a$-, un groupe représenté par RNH-CO-, un groupe représenté par $R_2$N-CO-, un groupe représenté par RO-CO-NR$^a$-, un groupe représenté par RNH-CO-NH-, un groupe représenté par $R_2$N-CO-NH-, un groupe représenté par RNH-CO-CO-NH-, un groupe représenté par $R_2$N-CO-CO-NH-, un groupe représenté par R-S(O)$_2$-NH-, un groupe représenté par $R_2$N-S(O)$_2$-, un groupe représenté par $R_2$S(O)=N-, un groupe représenté par R-S(O)(=N-R$^b$)-, un groupe représenté par RO-N=C(R$^c$)-, un groupe nitro ou un groupe cyano ;
chaque R représente indépendamment un groupe alkyle en $C_{1-6}$ substitué ou non substitué ou un groupe cycloalkyle en $C_{3-6}$ substitué ou non substitué,
chaque R$^a$ représente indépendamment un atome d'hydrogène, un groupe alkyle en $C_{1-6}$ substitué ou non substitué, ou un groupe alcoxy en $C_{1-6}$ substitué ou non substitué,
R$^b$ représente un groupe alkyle en $C_{1-6}$ substitué ou non substitué ou un groupe phényle substitué ou non substitué,
R$^c$ représente un atome d'hydrogène ou un groupe alkyle en $C_{1-6}$ substitué ou non substitué ;
dans le groupe ci-dessus représenté par $R_2$N-CO-, le groupe représenté par $R_2$N-CO-NH-, le groupe représenté par $R_2$N-CO-CO-NH- ou le groupe représenté par $R_2$N-S(O)$_2$-, R et R peuvent être liés pour former un cycle de 4 à 6 chaînons conjointement avec un atome d'azote auquel ils sont liés ;
dans le groupe ci-dessus représenté par $R_2$S(O)=N-, R et R peuvent être liés pour former un cycle de 5 à 6 chaînons conjointement avec un atome de soufre auquel ils sont liés ;
n représente un nombre entier de 0 à 5, lorsque n est 2 ou plus, les groupes X peuvent être identiques ou différents, et lorsque n est 2 ou plus, deux de leurs groupes X peuvent être combinés pour former un groupe organique divalent.

3. Composé représenté par la formule (I) selon la revendication 1 qui est représenté par une formule (I-3), ou sel de celui-ci :

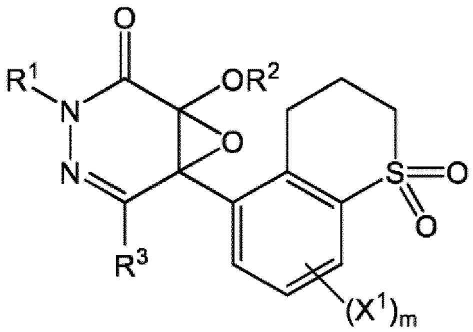

(I-3)

dans laquelle $R^1$, $R^2$ et $R^3$ sont identiques à ceux définis dans la revendication 1, dans laquelle $X^1$ représente un groupe halogéno, un groupe alkyle en $C_{1-6}$ substitué ou non substitué, un groupe alcényle en $C_{2-6}$ substitué ou non substitué, un groupe alcynyle en $C_{2-6}$ substitué ou non substitué, un groupe alcoxy en $C_{1-6}$ substitué ou non substitué, un groupe alkylthio en $C_{1-6}$ substitué ou non substitué, un groupe alkylsulfinyle en $C_{1-6}$ substitué ou non substitué, un groupe alkylsulfonyle en $C_{1-6}$ substitué ou non substitué, un groupe cycloalkyle en $C_{3-6}$ substitué ou non substitué, un groupe phényle substitué ou non substitué, un groupe hétérocyclyle de 5 à 6 chaînons substitué ou non substitué, un groupe nitro ou un groupe cyano ;
m représente un nombre entier de 0 à 3, et lorsque m est 2 ou plus, les groupes $X^1$ peuvent être identiques ou différents.

**4.** Composé selon la revendication 1, ou sel de celui-ci,

dans lequel un substituant sur le groupe alkyle en $C_{1-6}$ représenté par $R^2$ est au moins un substituant choisi dans le groupe constitué par un groupe halogéno, un groupe hydroxyle, un groupe alcoxy en $C_{1-6}$, un groupe alcoxy en $C_{1-6}$ alcoxy en $C_{1-6}$, un groupe halogénoalcoxy en $C_{1-6}$, un groupe alkylthio en $C_{1-6}$, un groupe alkylsulfinyle en $C_{1-6}$, un groupe alkylsulfonyle en $C_{1-6}$, un groupe cycloalkyle en $C_{3-6}$, un groupe phényle, un groupe hétéroaryle à 5 chaînons, un groupe alkylcarbonyle en $C_{1-6}$, un groupe benzoyle, un groupe alcoxycarbonyle en $C_{1-6}$, un groupe alkylcarboxamide en $C_{1-6}$, un groupe (1,3-dioxoisoindrin-2-yl)oxy, un groupe triméthylsilyle et un groupe cyano, et
un groupe constitué par un groupe alkyle en $C_{1-6}$ substitué, un groupe halogéno substitué, un groupe halogénoalkyle en $C_{1-6}$ substitué ou un groupe phényle subs-titué par un groupe halogénoalcoxy en $C_{1-6}$ ; un groupe alkyle en $C_{1-6}$ substitué, un groupe halogéno substitué, un groupe halogénoalkyle en $C_{1-6}$ substitué ou un groupe phénoxy substitué par un groupe halogénoalcoxy en $C_{1-6}$ ; et un groupe alkyle en $C_{1-6}$ substitué, un groupe halogéno substitué, un groupe halogénoalkyle en $C_{1-6}$ substitué ou un groupe hétéroaryle de 5 chaînons substitué par un groupe halogénoalcoxy en C1-6.

**5.** Herbicide comprenant au moins un herbicide choisi dans le groupe constitué du composé selon l'une quelconque des revendications 1 à 4 et d'un sel de celui-ci comme principe actif.

**6.** Procédé de lutte contre les mauvaises herbes monocotylédones et/ou dicotylédones chez les plantes utiles, qui est un procédé comprenant une étape d'application du composé selon l'une quelconque des revendications 1 à 4 ou d'un sel de celui-ci, ou d'un herbicide contenant ledit composé à ladite mauvaise herbe et/ou à ladite plante et/ou à son emplacement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019174531 A
- WO 2013050421 A
- EP 3438095 A1
- US 20170096402 A1